(19) Europäisches Patentamt
European Patent Office
Office européen des brevets

(11) EP 3 773 931 B1

(12) **FASCICULE DE BREVET EUROPEEN**

(45) Date de publication et mention de la délivrance du brevet:
**04.06.2025 Bulletin 2025/23**

(21) Numéro de dépôt: **19726044.1**

(22) Date de dépôt: **10.04.2019**

(51) Classification Internationale des Brevets (IPC):
***A61Q 19/00*** *(2006.01)* ***A61P 17/16*** *(2006.01)*
***A61K 8/37*** *(2006.01)* ***A61K 8/9789*** *(2017.01)*
***A61K 36/28*** *(2006.01)*

(52) Classification Coopérative des Brevets (CPC):
**A61Q 19/007; A61K 8/375; A61K 8/9789; A61K 36/28; A61P 17/16;** A61K 2236/00; A61K 2800/805; Y02P 60/21

(86) Numéro de dépôt international:
**PCT/FR2019/050840**

(87) Numéro de publication internationale:
**WO 2019/197777 (17.10.2019 Gazette 2019/42)**

(54) **NOUVELLE COMPOSITION À BASE D’ACIDES POLYCAFÉOYLQUINIQUES, SON UTILISATION EN COSMÉTIQUE ET COMPOSITIONS COSMÉTIQUES EN COMPRENANT**

NEUE ZUSAMMENSETZUNG ENTHALTEND POLYCAFEOYLQUININSÄUREN, IHRE KOSMETISCHE VERWENDUNG UND SIE ENTHALTENDE KOSMETISCHE ZUSAMMENSEZUNGEN

NOVEL COMPOSITION COMPRISING POLYCAFEOYLQUINIC ACIDS, COSMETIC USE THEREOF AND COSMETIC COMPOSITIONS COMPRISING IT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2018 FR 1853224**

(43) Date de publication de la demande:
**17.02.2021 Bulletin 2021/07**

(73) Titulaire: **Société d’Exploitation de Produits pour les Industries Chimiques SEPPIC**
**75321 Paris cedex 07 (FR)**

(72) Inventeurs:
- **KERN, Catherine**
  **75321 PARIS CEDEX 07 (FR)**
- **GARCIA, Christine**
  **81100 CASTRES (FR)**
- **GOMBERT, Christian**
  **75321 PARIS CEDEX 07 (FR)**
- **MSIKA, Philippe**
  **PARIS CEDEX 07 PARIS (FR)**

(74) Mandataire: **Air Liquide**
**L’Air Liquide S.A.**
**Direction de la Propriété Intellectuelle**
**75, Quai d’Orsay**
**75321 Paris Cedex 07 (FR)**

(56) Documents cités:
**WO-A1-2017/129779 US-B1- 8 481 480**

- **"Burdock Serum with Bh Intensiv + Complex", GNPD; MINTEL, June 2011 (2011-06-01), XP002717580**
- **AMOREPACIFIC: "Ice UV Sun Shot SPF 30 PA+ +", GNPD, MINTEL, 1 April 2014 (2014-04-01), XP002762462**
- **DATABASE WPI Week 201708, 2016 Derwent World Patents Index; AN 2016-72299D, XP002786839**
- **"Dictionnary International Cosmetic Ingredient Handbook / Natural Solution", INTERNATIONAL COSMETIC INGREDIENT DICTIONARY AND HANDBOOK, XX, XX, 25 October 2005 (2005-10-25), pages 1 - 9(0133), XP002443847**

Il est rappelé que: Dans un délai de neuf mois à compter de la publication de la mention de la délivrance du brevet européen au Bulletin européen des brevets, toute personne peut faire opposition à ce brevet auprès de l’Office européen des brevets, conformément au règlement d’exécution. L’opposition n’est réputée formée qu’après le paiement de la taxe d’opposition. (Art. 99(1) Convention sur le brevet européen).

- ANITA L. HAYDEN: "Aeroponic and Hydroponic Systems for Medicinal Herb, Rhizome, and Root Crops", HORTSCIENCE VOL. 41(3), 1 January 2006 (2006-01-01), pages 536 - 538, XP055525922, Retrieved from the Internet <URL:http://hortsci.ashspublications.org/content/41/3/536.full.pdf> [retrieved on 20181122]

## Description

La présente invention a pour objet un procédé de préparation pour une composition comprenant des dérivés d'acides quiniques poly-substitués, et plus particulièrement d'un extrait de la plante *Arctium lappa* comprenant lesdits dérivés. L'utilisation de ladite composition comprenant des dérivés d'acides quiniques poly-substitués pour préparer des formulations à usage topique destinées à hydrater la peau, et plus particulièrement l'épiderme de la peau humaine ou animale est décrite à titre illustratif dans la présente demande.

L'invention trouve principalement application dans les domaines cosmétiques et dermopharmaciques, mais également dans le domaine de l'industrie textile par exemple pour le traitement de fibres textiles synthétiques ou naturelles tissées ou tricotées, ou encore dans le domaine de l'industrie papetière par exemple pour la fabrication de papier à usage sanitaire ou domestique.

La peau est un organe atypique du corps humain, extrêmement fin au regard de son étendue mais également l'organe le plus lourd d'un individu. Une des caractéristiques de la peau réside dans le fait qu'il s'agit d'un organe d'interface, d'un organe limite, entre le milieu intérieur (corps humain) et le milieu extérieur. De ce fait, et avec la flore qui la recouvre et l'habite, la peau est la première barrière de protection de l'organisme humain.

En raison de sa position d'interface avec le milieu extérieur, la peau est soumise à des sollicitations quotidiennes nombreuses, telles que par exemple le contact avec des vêtements, les changements de températures, les changements de degrés d'hygrométrie, les changements de pression, voire à des agressions, comme par exemple le contact avec certains produits chimiques présentant ou pouvant présenter un caractère très acide, ou très basique, ou irritant, avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui.

Par conséquent, l'amélioration de son aspect est un sujet de préoccupation constant pour les êtres humains. La peau est le reflet d'un état de bien-être, souvent associé à la jeunesse, et *a contrario* d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.

A la périphérie de l'épiderme, se trouve une couche cornée supérieure, nommée le *stratum corneum,* qui est la première couche de l'épiderme à subir les stress d'origine externe, comme les variations de conditions climatiques extérieures (température, pression, hygrométrie) ou les sollicitations mécaniques.

Le *stratum corneum* est plus particulièrement en contact avec le microbiote cutané.

Par « microbiote cutané », on désigne au sens de la présente demande une population de micro-organismes, spécialisés ou opportunistes, comme par exemple les bactéries, les champignons, les levures..., qui vit à la surface de la peau.

Le microbiote cutané ne peut pas être défini de façon spécifique et généralisé pour l'ensemble des individus. Depuis le lancement en 2007 du projet « Human Microbiome Project » (HMP) du National Institute of Health, les chercheurs ont pu observer de grandes variations topographiques du microbiote humain ainsi que de grandes différences selon les individus.

Au moins dix-neuf *phyla* ont été identifiés dont les quatre principaux sont Actinobacteria (51,8%), Firmicutes (24,4%), Proteobacteria (16,5%) et Bacteroidetes (6,3%). Les genres majoritairement identifiés sont *Corynebacterium, Propionibacterium* et *Staphylococcus.* L'abondance de chaque groupe est fortement dépendante des différentes localisations. Les organismes fongiques isolés sur la peau sont du genre *Malassezia* spp. Par ailleurs, des acariens du genre Demodex sont également présents et résident dans les unités pilo-sébacées, le plus souvent de la surface du visage.

Ce microbiote s'alimente à la fois de molécules excrétées par la peau (lipides, protéines...), et de composés sécrétés par les communautés de micro-organismes mettant en évidence une réelle coopération au sein de ce microbiote. De plus, cette relation avec l'hôte constitue une véritable symbiose.

Les bactéries peuvent être commensales lorsqu'elles vivent au contact du revêtement cutanéo-muqueux d'un hôte sans entraîner de dommages. Un équilibre s'installe alors entre l'individu et les flores diverses commensales de la peau et des muqueuses, mais cet équilibre est sans cesse menacé par les agressions physiques ou chimiques subies par le *stratum corneum,* comme par exemple la pollution, les variations de température, les rayonnements ultra-violet, l'utilisation intensive de produits tensioactifs détergents, le stress, etc.... A côté de ces bactéries commensales, se trouvent les bactéries opportunistes, transitoires, indésirables et/ou pathogènes. *Staphylococcus epidermidis (S. epidermidis)* constitue plus de 90% de la flore résidente en aérobie présente dans le *stratum corneum.* La flore résidente est aussi composée de bactéries anaérobie appartenant à la division des Actinobactéries comme *Propionibacterium acnes (P. acnes),* fréquemment retrouvés au niveau des zones sébacées, comme par exemple le dos, le visage et le cuir chevelu.

Alors que la flore normale de la peau constitue une défense pour l'hôte, une augmentation ou une réduction de la composition bactérienne (dysbiose) peut conduire à un déséquilibre physiologique, comme par exemple, en ralentissant la différenciation des kératinocytes contenus dans l'épiderme ce qui provoque un affaiblissement de la fonction barrière de la peau, et par conséquent, rendre le *stratum corneum* plus vulnérable à la déshydratation et à la sécheresse.

C'est pourquoi, l'amélioration de l'apparence ou le maintien de la bonne apparence de la peau humaine, consiste notamment à maintenir ou renforcer la fonction barrière de l'épiderme de façon à conserver ou à atteindre un état d'hydratation du *stratum corneum à* un niveau optimal et satisfaisant. Cela permet en plus d'éviter les inconvénients esthétiques et physiologiques liés à un état de sécheresse de la peau.

De nombreuses solutions ont déjà été apportées pour résoudre les problèmes de sécheresse de la peau dues à une déshydratation du *stratum corneum,* notamment par la mise au point de compositions hydratantes.

Une amélioration ou une préservation de l'hydratation de la peau, et plus particulièrement du *stratum corneum,* peut se concevoir par mise en contact de la peau avec des composés qui possèdent un « effet hydratant ».

Par « effet hydratant », on entend une augmentation du taux d'hydratation du *stratum corneum* résultant de l'application topique d'une substance chimique ou d'une composition chimique sur la surface de la peau à traiter.

Comme agent hydratant de la peau, et plus particulièrement des peaux très sèches et déstructurées, il y a :

- Les agents occlusifs qui se caractérisent par leur aptitude à former un film imperméable à la surface de la peau et à diminuer ainsi fortement l'évaporation de l'eau à la surface de l'épiderme. De tels agents sont par exemple les huiles minérales comme la vaseline ou les paraffines liquides, la glycérine, le beurre de karité (Butyrospermum parkii butter), la cire d'abeille (Cera alba), certaines huiles végétales comme l'huile de germe de blé, l'huile de coco, le beurre de cacao, la lanoline et les dérivés de la silicone ;
- Les agents émollients qui se caractérisent par leur aptitude à combler les espaces intercellulaires se trouvant entre les cornéocytes (cellules de la couche cornée de l'épiderme) ; ils limitent aussi de l'évaporation de l'eau de l'épiderme, mais dans une moindre mesure que les agents occlusifs. De tels agents sont par exemple les céramides, l'acide linoléique et certaines huiles végétales comme l'huile d'amande douce ou l'huile de jojoba ;
- Les agents filmogènes, qui se caractérisent par leur aptitude à s'associer à l'eau pour former des hydrogels semi-perméables ; ils participent ainsi à la modulation de l'évaporation de l'eau du *stratum corneum.* De tels agents sont par exemple, le collagène, l'élastine, l'ADN, la pectine, la gélatine, le chitosan, ou les glycosaminoglycans comme l'acide hyaluronique ;
- Les agents humectants, substances hydrophiles et qui se caractérisent par leur fort pouvoir hygroscopique, à savoir leur aptitude à retenir l'eau. Ils contribuent ainsi à permettre au *stratum corneum* de conserver à la fois l'eau qu'il renferme, et celle apportée par la formule cosmétique. de tels agents sont par exemple l'urée, le glycérol, l'acide lactique, les acides aminés, le lactate de sodium, le propylène de glycol, les polyéthylèneglycols, les acides alpha-hydroxylés ou le sorbitol. Le glycérol, l'urée et l'acide lactique sont les agents humectants les plus utilisés dans les compositions cosmétiques hydratantes, et tout particulièrement le glycérol pour son coût très compétitif.

Cependant, certains agents humectants, comme la glycérine, ont aussi un effet occlusif immédiat, ce qui n'est pas désiré pour les peaux normales dont la fonction barrière n'est pas défaillante. De plus certains d'entre eux, comme la glycérine, provoquent certaines irritations de la peau et des muqueuses chez des personnes particulièrement sensibles.

Parmi les extraits de végétaux que l'on peut utiliser pour leurs actions sur le microbiote humain, on peut citer un extrait lyophilisé de feuilles de bardane (ou *Arctium lappa*) pour lequel une activité antibactérienne a été mise en évidence et plus particulièrement une activité contre des micro-organismes oraux, se montrant plus efficaces contre des bactéries associés à des pathogènes endodontiques tels que *Bacillus subtilis, Candida albicans, Lactobacillus acidophilus* et *Pseudomonas aeruginosa (1).*

La demande de brevet chinois publiée sous le numéro CN 104304955 A, divulgue une composition complexe

obtenue par mélange de différentes parties de plantes, parmi lesquelles on trouve les racines de bardane, puis par centrifugation pour obtenir le jus de ce mélange complexe de parties de plantes végétales. Elle divulgue également que les herbes médicinales utilisées, comme les racines de bardane, sont utilisées à long terme pour leurs effets bénéfiques sur les reins, sur l'hydratation de la peau et pour retarder le vieillissement.

La demande de brevet chinois publiée sous le numéro CN105232438 A, divulgue un masque destiné à hydrater le visage, comprenant un mélange complexe dans lequel on trouve de nombreux extraits de racines parmi lesquels un extrait de racines de bardane.

La demande de brevet chinois publiée sous le numéro CN107157800 A divulgue une formulation complexe comprenant des polysaccharides issus de racines de bardane ayant un effet hydratant, antibactérien, anti-inflammatoire, anti-âge et antifatigue.

La demande de brevet chinois publiée sous le numéro CN106074663 A divulgue une composition d'extraits de plantes comprenant un extrait de bois de Milo, un extrait de racines de bardane, et un extrait de chèvrefeuille, et décrit plus particulièrement que l'extrait de racines de Bardane traite les peaux sèches, le prurit aigu, l'inflammation, les cicatrices et d'autres symptômes, en inhibant des facteurs inflammatoires induits par différentes causes (stress externe ou facteurs génétiques), améliorant l'activité immunitaire et l'activité anti-oxydante de la peau, apaisant et réparant la peau.

Le document « Dictionary International Cosmetic Ingredient Handbook / Natural Solution », INTERNATIONAL COSMETIC INGREDIENT DICTIONARY AND HANDBOOK, décrit un produit (page 5, No. 88) comprenant un extrait alcoolique de racine *d'Atrium Lappa* (Water (and) Butylene Glycol (and) *Atrium Lappa* Root Extract).

Dans le cadre de leurs recherches concernant l'amélioration de l'hydratation de la peau, les inventeurs se sont attachés à développer une nouvelle composition comprenant des acides quiniques poly-substitués (ou « QPS »). ayant des effets positifs sur l'hydratation de la peau humaine.

L'invention a pour objet un procédé pour la préparation d'une composition ($C_1$) comprenant pour 100% de sa masse :

**a)** - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;

**b)** - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

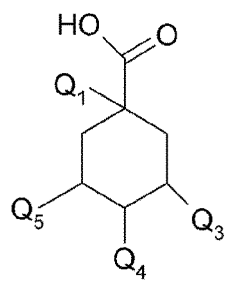

(I),

dans laquelle $Q_1$, $Q_3$, $Q_4$ et $Q_5$ représentent indépendamment les uns des autres, le radical hydroxyle ou un ses sels ou un radical choisi parmi :

**(i)** - Le radical caféoyle de formule (II) :

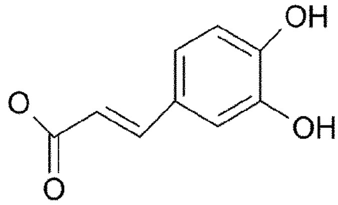

(II)

**(ii)** - Le radical maloyle de formule (IIIa) ou (IIIb) :

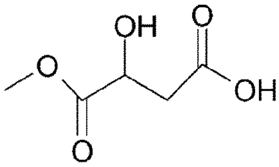
(IIIa)
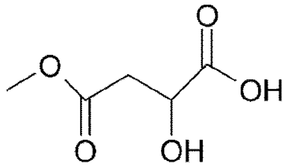
(IIIb) ;

**(iii)** - Le radical caféoyl maloyle de formule (IVa) ou (IVb) :

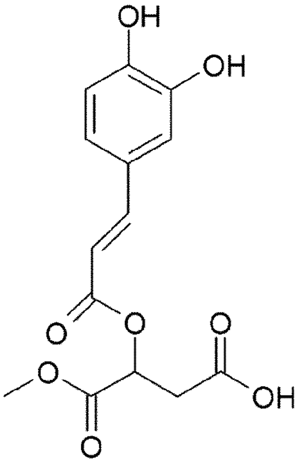
(IVa)
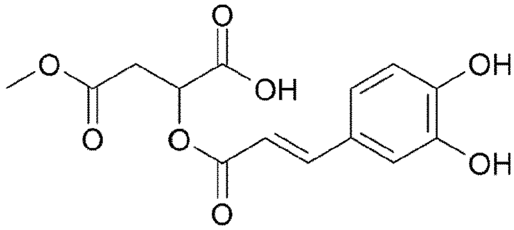

(IVb)

**(iv)** - Le radical maloyl caféoyle de formule (Va), (Vb), (Vc) ou (Vd),

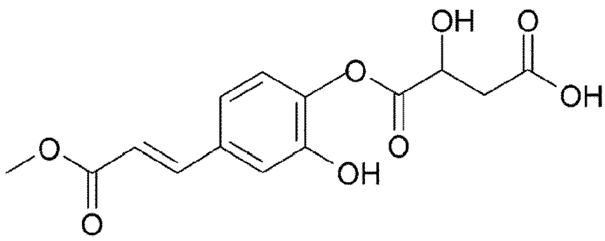
(Va)

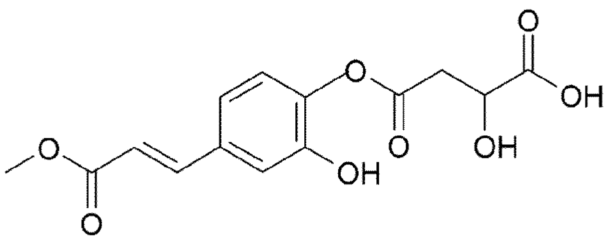
(Vb)

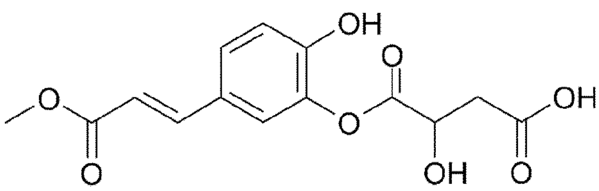
(Vc),

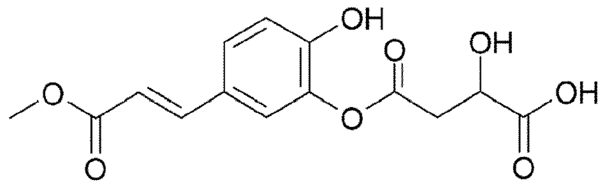

(Vd)

étant entendu qu'au moins un de ces radicaux $Q_1$, $Q_3$, $Q_4$ et $Q_5$ ne représente ni le radical -OH ; ni un de ses sels ; et

**c)** - De 20,0% massique à 35,0% massique d'eau, ledit procédé comprenant les étapes successive suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/milieu ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;
- Une étape c) de séparation des racines de la biomasse à l'issue du traitement défini à l'étape b), pour isoler une phase liquide ($L_1$) ;
- Une étape d) d'immersion des racines de ladite biomasse ($BM_2$) issue de l'étape c) dans ledit milieu ($S_1$); dans un ratio biomasse ($BM_2$)/milieu ($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$) ,
- Une étape f) de filtration de ladite phase liquide ($L_2$) obtenue à l'étape e), pour isoler une phase liquide ($L_3$),
- Une étape g) de mélange des dites phases liquides ($L_1$) et ($L_3$), puis si nécessaire d'addition d'eau et/ou dudit solvant organique ($SO_1$), de façon à obtenir la composition ($C_1$) attendue.

Au sens de la présente invention, l'expression «ladite quantité massique $x_1$ étant exprimée en équivalent massique de l'acide 1-O-(2-caféoyl)maloyl-3,5-O-dicaféoyl quinique » signifie que la quantité massique $x_1$ a été déterminée par la mise en œuvre d'une méthode analytique quantitative de type UHPLC-MS (« Ultra High Performance Liquid Chromatography-Mass Spectra), utilisant comme étalon de référence un standard de l'acide 1-O-(2-caféoyl) maloyl-3,5-O-dicaféoyl quinique préalablement isolé et purifié à une teneur supérieure ou égale à 99%.

Une telle analyse quantitative de type UHPLC-MS a été réalisée avec un appareillage de type UHPLC-MS Shimadzu_Nexera_LCMS 2020, équipé d'un détecteur à barrette de diode et réglé à une longueur d'onde de 330 nanomètres, d'une colonne de type Kinetex 2,6u XB-C18 100A. 100 x 2,1, et mettant en œuvre une phase mobile A composée d'eau et de 0,1% massique d'acide formique et une phase mobile B constituée par l'acétonitrile.

Parmi les composés de formule générale (I) présents dans la composition (ES), on peut citer :

- Les composés de la famille des acides DiCaféoylQuiniques (DCQ) tels que décrit dans le tableau 1 ci-dessous :

**Tableau 1**

| Acide DiCaféoylQuiniques (DCQ) | | | |
|---|---|---|---|
| **Acide 1,3-O-dicaféoylquinique (1,3-DCQ)** | | | |
| **Q1** | **Q3** | **Q4** | **Q5** |
| Caféoyl | Caféoyl | OH | OH |
| **Acide 1,4-O-dicaféoylquinique (1,4-DCQ)** | | | |
| **Q1** | **Q3** | **Q4** | **Q5** |
| Caféoyl | OH | Caféoyl | OH |
| **Acide 1,5-O-dicaféoylquinique (1,5-DCQ)** | | | |
| **Q1** | **Q3** | **Q4** | **Q5** |
| Caféoyl | OH | OH | Caféoyl |

(suite)

| Acide 3,4-O-dicaféoylquinique (3,4-DCQ) | | | |
|---|---|---|---|
| **Q1** | **Q3** | **Q4** | **Q5** |
| OH | Caféoyl | Caféoyl | OH |
| **Acide 3,5-O-dicaféoylquinique (3,5-DCQ)** | | | |
| **Q1** | **Q3** | **Q4** | **Q5** |
| OH | Caféoyl | OH | Caféoyl |
| **Acide 4,5-O-dicaféoylquinique (4,5 DCQ)** | | | |
| **Q1** | **Q3** | **Q4** | **Q5** |
| OH | OH | Caféoyl | Caféoyl |

- Les composés de la famille des acides TriCaféoylQuiniques (TCQ) tels que décrit dans le tableau 2 ci-dessous :

**Tableau 2**

| Acide TriCaféoylQuinique (TCQ) | | | |
|---|---|---|---|
| **Acide 1,3,4-O-tricaféoylquinique (1,3,4-TCQ)** | | | |
| **Q1** | **Q3** | **Q4** | **Q5** |
| Caféoyl | Caféoyl | Caféoyl | OH |
| **Acide 1,3,5-O-tricaféoylquinique (1,3,5-TCQ)** | | | |
| **Q1** | **Q3** | **Q4** | **Q5** |
| Caféoyl | Caféoyl | OH | Caféoyl |
| **Acide 1,4,5-O-tricaféoylquinique (1,4,5-TCQ)** | | | |
| **Q1** | **Q3** | **Q4** | **Q5** |
| Caféoyl | OH | Caféoyl | Caféoyl |
| **Acide 3,4,5-O-tricaféoylquinique (1,3,4-TCQ)** | | | |
| **Q1** | **Q3** | **Q4** | **Q5** |
| OH | Caféoyl | Caféoyl | Caféoyl |

- Les composés de la famille des acides Maloyl TriCaféoylQuiniques (m-TCQ) tels que décrit dans le tableau 3 ci-dessous :

**Tableau 3**

| Maloyl TriCaféoylQuiniques (m-TCQ) | | | |
|---|---|---|---|
| **Acide 1-O-maloyl-(3,4,5-O-tricaféoyl)quinique** | | | |
| **Q1** | **Q3** | **Q4** | **Q5** |
| Maloyl | Caféoyl | Caféoyl | Caféoyl |
| **Acide 3-O-maloyl-(3,4,5-O-tricaféoyl)quinique** | | | |
| **Q1** | **Q3** | **Q4** | z |
| Caféoyl | Maloyl | Caféoyl | Caféoyl |
| **Acide 4-O-maloyl-(1,3,5-O-tricaféoyl)quinique** | | | |
| **Q1** | **Q3** | **Q4** | **Q5** |
| Caféoyl | Caféoyl | Maloyl | Caféoyl |

(suite)

| Acide 5-O-maloyl-(1,3,4-O-tricaféoyl)quinique | | | |
|---|---|---|---|
| **Q1** | **Q3** | **Q4** | **Q5** |
| Caféoyl | Caféoyl | Caféoyl | Maloyl |

- Les composés de la famille des acides Maloyl DiCaféoylQuiniques (m-DCQ) tels que décrit dans le tableau 4 ci-dessous

**Tableau 4**

| **Acide Maloyl DiCaféoylQuinique (m-DCQ)** | | | |
|---|---|---|---|
| **Acide 4-O-maloyl- (1,3-O-dicaféoyl)quinique** | | | |
| **Q1** | **Q3** | **Q4** | **Q5** |
| Caféoyl | Caféoyl | Maloyl | OH |
| Acide 5-O-maloyl- (1,3-O-dicaféoyl)quinique | | | |
| Q1 | Q3 | Q4 | Q5 |
| Caféoyl | Caféoyl | OH | Maloyl |
| Acide 3-O-maloyl- (1,4-O-dicaféoyl)quinique | | | |
| Q1 | Q3 | Q4 | Q5 |
| Caféoyl | Maloyl | Caféoyl | OH |
| **Acide 5-O-maloyl- (1,4-O-dicaféoyl)quinique** | | | |
| **Q1** | **Q3** | **Q4** | **Q5** |
| Caféoyl | OH | Caféoyl | Maloyl |
| **Acide 3-O-maloyl- (1,5-O-dicaféoyl)quinique** | | | |
| **Q1** | **Q3** | **Q4** | **Q5** |
| Caféoyl | Maloyl | OH | Caféoyl |
| **Acide 4-O-maloyl- (1,5-O-dicaféoyl)quinique** | | | |
| **Q1** | **Q3** | **Q4** | **Q5** |
| Caféoyl | OH | Maloyl | Caféoyl |
| **Acide 1-O-maloyl- (3,4-O-dicaféoyl)quinique** | | | |
| **Q1** | **Q3** | **Q4** | **Q5** |
| Maloyl | Caféoyl | Caféoyl | OH |
| **Acide 5-O-maloyl- (3,4-O-dicaféoyl)quinique** | | | |
| **Q1** | **Q3** | **Q4** | **Q5** |
| OH | Caféoyl | Caféoyl | Maloyl |
| **Acide 1-O-maloyl- (3,5-O-dicaféoyl)quinique** | | | |
| **Q1** | **Q3** | **Q4** | **Q5** |
| Maloyl | Caféoyl | OH | Caféoyl |
| **Acide 4-O-maloyl- (3,5-O-dicaféoyl)quinique** | | | |
| **Q1** | **Q3** | **Q4** | **Q5** |
| OH | Caféoyl | Maloyl | Caféoyl |

(suite)

| Acide 3-O-maloyl- (4,5-O-dicaféoyl)quinique | | | |
|---|---|---|---|
| Q1 | Q3 | Q4 | Q5 |
| OH | Maloyl | Caféoyl | Caféoyl |
| OH | Maloyl | Caféoyl | Caféoyl |

- Les composés de la famille des acides CaféoylMaloyl TriCaféoylQuiniques (cm-TCQ) tels que décrit dans le tableau 5 ci-dessous :

**Tableau 5**

| Acide CaféoylMaloyl TriCaféoylQuinique (cm-TCQ) | | | |
|---|---|---|---|
| Acide 1-O--(2-caféoyl)maloyl -(3,4,5-O-tricaféoyl)quinique | | | |
| Q1 | Q3 | Q4 | Q5 |
| CaféoylMaloyl | Caféoyl | Caféoyl | Caféoyl |
| Acide 3-O--(2-caféoyl)maloyl-(1,4,5-O-tricaféoyl)quinique | | | |
| Q1 | Q3 | Q4 | Q5 |
| Caféoyl | CaféoylMaloyl | Caféoyl | Caféoyl |
| Acide 4-O--(2-caféoyl)maloyl-(1,3,5-O-tricaféoyl)quinique | | | |
| Q1 | Q3 | Q4 | Q5 |
| Caféoyl | Caféoyl | CaféoylMaloyl | Caféoyl |
| Acide 5-O-(2-caféoyl)maloyl-(1,3,4-O-tricaféoyl)quinique | | | |
| Q1 | Q3 | Q4 | Q5 |
| Caféoyl | Caféoyl | Caféoyl | CaféoylMaloyl |

- Les composés de la famille des acides CaféoylMaloyl DiCaféoylQuiniques (cm-DCQ) tels que décrit dans le tableau 6 ci-dessous

**Tableau 6**

| Acide CaféoylMaloyl DiCaféoylQuinique (cm-DCQ) | | | |
|---|---|---|---|
| Acide 4-O-(2-caféoyl)maloyl (1,3-O-dicaféoyl)quinique | | | |
| Q1 | Q3 | Q4 | Q5 |
| Caféoyl | Caféoyl | CaféoylMaloyl | OH |
| Acide 5-O-(2-caféoyl)maloyl (1,3-O-dicaféoyl)quinique | | | |
| Q1 | Q3 | Q4 | Q5 |
| Caféoyl | Caféoyl | OH | CaféoylMaloyl |
| Acide 3-O-(2-caféoyl)maloyl- (1,4-O-dicaféoyl)quinique | | | |
| Q1 | Q3 | Q4 | Q5 |
| Caféoyl | CaféoylMaloyl | Caféoyl | OH |
| Acide 5-O-(2-caféoyl)maloyl -(1,4-O-dicaféoyl)quinique | | | |
| Q1 | Q3 | Q4 | Q5 |
| Caféoyl | OH | Caféoyl | CaféoylMaloyl |

(suite)

| Acide 3-O-(2-caféoyl)maloyl - (1,5-O-dicaféoyl)quinique | | | |
|---|---|---|---|
| **Q1** | **Q3** | **Q4** | **Q5** |
| Caféoyl | CaféoylMaloyl | OH | Caféoyl |
| **Acide 4-O-(2-caféoyl)maloyl - (1,5-O-dicaféoyl)quinique** | | | |
| **Q1** | **Q3** | **Q4** | **Q5** |
| Caféoyl | OH | CaféoylMaloyl | Caféoyl |
| **Acide 1-O-(2-caféoyl)maloyl - (3,4-O-dicaféoyl)quinique** | | | |
| **Q1** | **Q3** | **Q4** | **Q5** |
| CaféoylMaloyl | Caféoyl | Caféoyl | OH |
| **Acide 5-O-(2-caféoyl)maloyl - (3,4-O-dicaféoyl)quinique** | | | |
| **Q1** | **Q3** | **Q4** | **Q5** |
| OH | Caféoyl | Caféoyl | CaféoylMaloyl |
| **Acide 1-O-(2-caféoyl)maloyl - (3,5-O-dicaféoyl)quinique** | | | |
| **Q1** | **Q3** | **Q4** | **Q5** |
| Maloyl | Caféoyl | OH | Caféoyl |
| **Acide 4-O-(2-caféoyl)maloyl - (3,5-O-dicaféoyl)quinique** | | | |
| **Q1** | **Q3** | **Q4** | **Q5** |
| OH | Caféoyl | CaféoylMaloyl | Caféoyl |
| **Acide 1-O-(2-caféoyl)maloyl - (4,5-O-dicaféoyl)quinique** | | | |
| **Q1** | **Q3** | **Q4** | **Q5** |
| CaféoylMaloyl | OH | Caféoyl | Caféoyl |
| **Acide 3-O-(2-caféoyl)maloyl - (4,5-O-dicaféoyl)quinique** | | | |
| **Q1** | **Q3** | **Q4** | **Q5** |
| OH | CaféoylMaloyl | Caféoyl | Caféoyl |

Selon un aspect plus particulier de la présente invention, dans la composition (ES) telle que définie précédemment, le composé de formule (Ia) correspondant à la formule (I) telle que définie précédemment et pour laquelle $Q_1$ représente le radical maloyl de formule (IIIb), $Q_3$ et $Q_4$ et $Q_5$, identiques, représentent le radical caféoyl de formule (II).

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVa) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ib) correspondant à la formule (I) telle que définie précédemment et pour laquelle $Q_1$ représente le radical caféoylmaloyl de formule (IVb) ; $Q_3$ et $Q_5$, identiques, représentent le radical caféoyl de formule (II) ; $Q_4$ représente le radical -OH.

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$ identiques, représentent le radical caféoyl de formule (II) ; $Q_3$ représente le radical -OH ; $Q_4$ représente le radical caféoylmaloyl de formule (IVa).

Selon un aspect plus particulier de la présente invention, dans la composition (ES) telle que définie précédemment, le composé de formule (Ic) est le composé de formule ($Ic_1$), correspondant à la formule (I) telle que définie précédemment et pour laquelle $Q_1$ et $Q_5$, identiques, représentent le radical caféoyl de formule (II) ; $Q_3$ représente le radical -OH ; $Q_4$ représente le radical caféoylmaloyl de formule (IVb).

Selon un aspect plus particulier de la présente invention, dans la composition (ES) telle que définie précédemment, le composé de formule (Ic) est le composé de formule ($Ic_2$), correspondant à la formule (I) telle que définie

précédemment et pour laquelle $Q_1$ et $Q_4$ identiques, représentent le radical caféoyl de formule (II) ; $Q_3$ représente le radical -OH ; $Q_5$ représente le radical caféoylmaloyl de formule (IVa).

Selon un aspect plus particulier de la présente invention, dans la composition (ES) telle que définie précédemment, le composé de formule (Ic) est le composé de formule ($Ic_2$), correspondant à la formule (I) telle que définie précédemment et pour laquelle $Q_1$ et $Q_4$ identiques, représentent le radical caféoyl de formule (II) ; $Q_3$ représente le radical -OH ; $Q_5$ représente le radical caféoylmaloyl de formule (IVb).

Selon un aspect plus particulier de la présente invention, la composition ($C_1$) telle que définie précédemment est caractérisée en ce que ladite composition (ES) comprend :

- Au moins un composé de formule (Ia) correspondant à la formule (I) pour laquelle $Q_1$ représente le radical maloyle de formule (IIIa) ou de formule (IIIb) et $Q_3$ et $Q_4$ et $Q_5$ identiques, représentent chacun le radical caféoyle de formule (II) ;
- Un composé de formule (Ib) correspondant à la formule (I) pour laquelle $Q_1$ représente le radical caféoylmaloyle de formule (IVa) ou de formule (IVb), $Q_3$ et $Q_5$ représentent chacun le radical caféoyle de formule (II) et $Q_4$ représente le radical hydroxyle, et
- Au moins un composé de formule (Ic) choisi parmi :
    - Le composé de formule ($Ic_1$) correspondant à la formule (I) pour laquelle $Q_1$ et $Q_5$ représentent chacun le radical caféoyle de formule (II), $Q_3$ représente le radical hydroxyle et $Q_4$ représente le radical caféoylmaloyle de formule (IVa) ou de formule (IVb) ; et
    - Le composé de formule ($Ic_3$) correspondant à la formule (I) pour laquelle $Q_1$ et $Q_4$ représentent le radical caféoyle de formule (II), $Q_3$ représente le radical hydroxyle et $Q_5$ représente le radical caféoylmaloyle de formule (IVa) ou de formule (IVb).

Selon un autre aspect particulier de la présente invention, le solvant organique ($SO_1$) présent dans la composition ($C_1$) telle que définie précédemment est choisi parmi le 1,2-propanediol, le 1,3-propanediol, et le 2-méthyl-2,4-pentanediol ; il s'agit plus particulièrement 1,2-propanediol.

L'invention a pour objet un procédé pour la préparation de la composition ($C_1$) comprenant les étapes successives suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/milieu ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;
- Une étape c) de séparation des racines de la biomasse à l'issue du traitement défini à l'étape b), pour isoler une phase liquide ($L_1$) ;
- Une étape d) d'immersion des racines de ladite biomasse ($BM_2$) issue de l'étape c) dans ledit milieu ($S_1$); dans un ratio biomasse ($BM_2$)/milieu($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$) ,
- Une étape f) de filtration de ladite phase liquide ($L_2$) obtenue à l'étape e), pour isoler une phase liquide ($L_3$),
- Une étape g) de mélange des dites phases liquides ($L_1$) et ($L_3$), puis si nécessaire d'addition d'eau et/ou dudit solvant organique ($SO_1$), de façon à obtenir la composition ($C_1$) attendue.

L'étape a) de mise en culture en conditions hors sol (ou dite en aéroponie) de la plante *Arctium lappa* se réalise selon les conditions standard connues de l'homme du métier, et plus particulièrement celles concernant l'influence de la teneur en azote (2) (3) (4) (5) (6), et de la teneur en phosphore et en potassium présentes dans le milieu de culture. L'étape a) de mise en culture en conditions hors sol se réalise donc en optimisant le ratio azote/phosphore/potassium présent dans le milieu nutritif, et en optimisant le paramètre d'électroconductivité d'un tel milieu nutritif.

L'étape a) est généralement conduite à une température comprise entre 20°C et 40°C, pendant une durée comprise entre 4 et 10 semaines, de façon à obtenir une biomasse ($BM_1$), en quantité importante notamment au niveau racinaire ; l'étape a) est arrêtée lorsque la croissance de la biomasse ($BM_1$) n'est plus observée.

Selon un autre aspect particulier, lors de l'étape b) du procédé tel que défini précédemment, le milieu ($S_1$) comprenant pour 100% de sa propre masse, de 23% massique à 32% massique et encore plus particulièrement de 26%

massique à 32 % massique d'eau, et de 68% massique à 77% massique et encore plus particulièrement de 68% massique à 74% massique d'un solvant organique ($SO_1$) sélectionné parmi les éléments du groupe constitué par le 1,2-propanediol, le 1,3-propanediol, le 1-4-butanediol, le 1,3-butanediol, le 2-méthyl-2,4-pentanediol, le 1,6-hexanediol, le 1,8-octanediol.

Dans l'étape b) du procédé de préparation de la composition ($C_1$) telle que définie précédemment, le pH de l'eau présente dans le milieu ($S_1$) est ajusté à une valeur comprise entre 1,5 et 3,5 par ajout d'un acide protique choisi parmi les éléments du groupe constitué par l'acide sulfurique, l'acide phosphorique et l'acide chlorhydrique.

Selon un aspect plus particulier lors de l'étape b) du procédé tel que défini précédemment, le pH de l'eau présente dans le milieu ($S_1$) est amené à une valeur comprise entre 1,5 et 3,0, et encore plus particulièrement entre 1,5 et 2,5 par ajout d'un acide protique choisi parmi les éléments du groupe constitué par l'acide sulfurique, l'acide phosphorique et l'acide chlorhydrique.

Selon un aspect encore plus particulier lors de l'étape b) du procédé tel que défini précédemment, le pH de l'eau présente dans le milieu ($S_1$) est amené à une valeur comprise entre 1,5 et 2,5 par ajout de l'acide chlorhydrique.

Dans l'étape b) du procédé de préparation de la composition ($C_1$) telle que définie précédemment, la mise en contact des racines de la biomasse ($BM_1$) obtenue à l'issue de l'étape a) avec le milieu ($S_1$) tel que précédemment décrit est réalisée pendant une durée comprise entre 10 minutes et 60 minutes, à une température comprise entre 20°C et 30°C, avec un ratio biomasse ($BM_1$)/milieu (S1) compris entre 0,5 kg/L et 1,5 kg/L.

Selon un aspect plus particulier lors de l'étape b) du procédé tel que défini précédemment, la mise en contact des racines de la biomasse ($BM_1$) obtenue à l'issue de l'étape a) avec le milieu ($S_1$), plus particulièrement par immersion, tel que précédemment décrit, est réalisée pendant une durée comprise entre 10 minutes et 45 minutes, plus particulièrement entre 10 minutes et 30 minutes, à une température comprise entre 20°C et 30°C, avec un ratio biomasse ($BM_1$)/milieu (S1) compris entre 0,5 kg/L et 1,5 kg/L , plus particulièrement compris entre 0,5 kg/L et 1,0 kg/L.

Dans l'étape d) du procédé de préparation de la composition ($C_1$) telle que définie précédemment, la mise en contact de la biomasse ($BM_2$) obtenue à l'issue de l'étape c) avec le milieu ($S_1$) tel que précédemment décrit est réalisée pendant une durée comprise entre 24 heures et 72 heures, à une température comprise entre 20°C et 30°C, avec un ratio biomasse ($BM_2$)/milieu ($S_1$) compris entre 0,1 kg/L et 1,5 kg/L.

Selon un aspect plus particulier lors de l'étape d) du procédé tel que défini précédemment, la mise en contact des racines de la biomasse ($BM_2$) obtenue à l'issue de l'étape c) avec le milieu ($S_1$), plus particulièrement par immersion, tel que précédemment décrit, est réalisée pendant une durée comprise entre 24 heures et 60 heures, plus particulièrement entre 24 heures et 48 heures, à une température comprise entre 20°C et 30°C, avec un ratio biomasse ($BM_2$)/milieu ($S_1$) compris entre 0,1 kg/L et 1,0 kg/L, et plus particulièrement entre 0,3 kg/L et 1,0 kg/L.

Dans l'étape f) du procédé de préparation de la composition ($C_1$) telle que définie précédemment, la filtration du liquide ($L_2$) obtenu à l'issue de l'étape e), mise en œuvre pour récupérer un liquide ($L_3$), se réalise avec les dispositifs et matériels connus de l'homme du métier pour séparer de façon efficace les solides de liquides.

Dans l'étape g) du procédé de préparation de la composition ($C_1$) telle que définie précédemment, le mélange du liquide ($L_1$) obtenu à l'issue de l'étape c) et du liquide ($L_3$) obtenu à l'issue de l'étape f), se réalise à une température comprise entre 20°C et 30°C, sous agitation mécanique de type ancre à une vitesse comprise entre 50 tours/minute et 150 tours/minute. Si nécessaire, un ajout optionnel d'eau ou de solvant organique ($SO_1$) est réalisé de façon à obtenir la composition ($C_1$) attendue.

Selon un aspect particulier, lors de l'étape g) du procédé tel que défini précédemment, la teneur en solvant organique ($SO_1$) est ajustée entre 65% à 75% massique et la teneur en eau est ajustée 25% à 35% massique, plus particulièrement par ajout d'eau et/ou de solvant organique ($SO_1$)

Selon un autre aspect particulier de la présente invention, le solvant organique ($SO_1$) sélectionné parmi les éléments du groupe constitué par le 1,2-propanediol, le 1,3-propanediol, et le 2-méthyl-2,4-pentanediol.

Selon un autre aspect plus particulier de la présente invention, l'acide protique présent dans l'eau du milieu ($S_1$) mis en œuvre dans l'étape b) et dans l'étape d) du procédé de préparation de ladite composition ($C_1$) est l'acide phosphorique.

L'utilisation de la composition ($C_1$) telle que définie précédemment, comme agent actif cosmétique hydratant est aussi décrit à titre illustratif.

Une formulation cosmétique à usage topique comprenant au moins un excipient cosmétiquement acceptable et une quantité efficace de la composition ($C_1$) telle que définie précédemment est décrite à titre illustratif.

L'expression "à usage topique" utilisée dans la définition de la formulation cosmétique mise en œuvre dans le procédé cosmétique objet de la présente invention, signifie que ladite formulation est mise en œuvre par application sur la peau, qu'il s'agisse d'une application directe dans le cas d'une formulation cosmétique ou d'une application indirecte lorsque la formulation cosmétique selon l'invention est imprégnée sur un support destiné à être mis en contact avec la peau (papier, lingette, textile, dispositif transdermique, etc...).

Ladite formulation est généralement étalée sur la surface de la peau à traiter, puis la peau est massée quelques instants.

L'expression « cosmétiquement acceptable » utilisée dans la définition de la formulation cosmétique à usage

topique, mise en œuvre dans le procédé cosmétique objet de la présente invention, signifie selon la directive du Conseil de la Communauté Economique Européenne N°76/768/CEE du 27 juillet 1976 modifiée par la directive N°93/35/CEE du 14 juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par quantité efficace d'une composition ($C_1$) telle que définie précédemment et présente dans la formulation cosmétique à usage topique mise en œuvre dans le procédé cosmétique objet de la présente invention, on entend pour 100% de la masse de ladite formulation cosmétique à usage topique, une proportion comprise entre 0,01% et 5% massique, plus particulièrement entre 0,01% et 3% massique, encore plus particulièrement entre 0,1% et 3% massique, et encore plus particulièrement entre 0,5% et 2% massique en composition ($C_1$).

Les formulations cosmétiques à usage topique mises en œuvre dans le procédé cosmétique objet de la présente invention, se présentent généralement sous forme de solutions aqueuses ou hydro-alcooliques ou hydro-glycoliques, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

Les formulations cosmétiques à usage topique mises en œuvre dans le procédé cosmétique objet de la présente invention, peuvent être conditionnées dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, la composition ($C_1$), présente dans les formulations cosmétiques à usage topique mises en œuvre dans le procédé cosmétique objet de la présente invention, est associée à des additifs chimiques habituellement mis en œuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer à ladite composition ($C_1$), on peut citer les tensioactifs moussants et/ou détergents anioniques, cationiques, amphotères ou non ioniques ;

- parmi les tensioactifs anioniques moussants et/ou détergents, il y a par exemple les sels de métaux alcalins, de métaux alcalino-terreux, d'ammonium, d'amines, ou d'aminoalcools d'alkylethersulfates, d'alkylsulfates, d'alkylamidoéthersulfates, d'alkylarylpolyéthersulfates, de monoglycérides sulfates, d'alpha-oléfinesulfonates, de paraffinessulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, de dérivés N-acylés d'acides gras.
- parmi les tensioactifs amphotères moussants et/ou détergents, il y a par exemple les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.
- parmi les tensioactifs cationiques moussants et/ou détergents, il y a par exemple les dérivés d'ammoniums quaternaires.
- parmi les tensioactifs non ioniques moussants, il y a par exemple les alkyl polyglycosides comportant un radical aliphatique, linéaire ou ramifié, saturé ou insaturé, et comportant de huit à seize atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1,12-dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à ladite composition ($C_1$), il y a les esters gras d'alkyl polyglycosides éventuellement alcoxylés, comme les esters de méthyl polyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl

tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer à ladite composition ($C_1$), il y a les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide-2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

$$CH_2=C(R_3)-C(=O)-[CH_2-CH_2-O]_n-R_4 \qquad (VIII)$$

dans laquelle $R_3$ représente un atome d'hydrogène ou un radical méthyle, R4 représente un radical alkyle linéaire ou ramifié comportant de huit à trente atomes de carbone et n représente un nombre supérieur ou égal à un et inférieur ou égal à cinquante.

Lesdits polymères peuvent se présenter sous la forme d'une solution, d'une suspension aqueuse, d'une émulsion eau-dans-huile, d'une émulsion huile-dans-eau, d'une poudre. Comme exemples de polymères du commerce, il y a ceux commercialisés sous les appellations SIMULGEL™EG, SIMULGEL™EPG, SEPIGEL™305, SIMULGEL™600, SIMULGEL™NS, SIMULGEL™INS100, SIMULGEL™FL, SIMULGEL™A, SIMULGEL™SMS 88, SEPINOV™EMT10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC2, ARISTOFLEX™HMB, COSMEDIA™SP, FLOCARE™ET25, FLOCARE™ET75, FLOCARE™ET26, FLOCARE™ET30, FLOCARE™ET58, FLOCARE™PSD30, VISCOLAM™AT64, VISCOLAM™AT100.

Comme autres exemples d'agents épaississants et/ou gélifiants que l'on peut associer à ladite composition ($C_1$), il y a :

- Les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, tels que les galactomannanes provenant de la gomme de cassia (DS = 1/5), de la gomme de caroube (DS = 1/4), de la gomme de tara (DS = 1/3), de la gomme de guar (DS = 1/2) ou de la gomme de fenugrec (DS = 1 ) ;
- Les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme arabique et de gomme de karaya, les glucosaminoglycanes ;
- La cellulose et ses dérivés la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à ladite composition ($C_1$), il y a les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples d'eaux thermales ou minérales que l'on peut associer à ladite composition ($C_1$), il y a les eaux

thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à ladite composition ($C_1$), il y a les xylènes sulfonates, les cumènes sulfonates, l'hexyl polyglucoside, le (2-éthyl hexyl) polyglucoside, le n-heptyl polyglucoside.

Comme exemples d'agents déodorants que l'on peut associer à ladite composition ($C_1$), il y a les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de solvants et de co-solvants que l'on peut associer à ladite composition ($C_1$), il y a l'eau, l'éthylène glycol, le propylène glycol, le butylène glycol, l'hexylène glycol, le diéthylène glycol, les alcools hydrosolubles tels que l'éthanol, l'isopropanol ou le butanol, les mélanges d'eau et desdits solvants.

Comme exemples d'huiles que l'on peut associer à ladite composition ($C_1$), il y a les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les iso-alcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à ladite composition ($C_1$), il y a la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de matières grasses que l'on peut associer à ladite composition ($C_1$), il y a les alcools gras saturés ou insaturés, linéaires ou ramifiés, comportant de huit à trente-six atomes de carbone, ou les acides gras saturés ou insaturés, linéaires ou ramifiés, comportant de huit à trente-six 36 atomes de carbone.

Comme exemples de filtres solaires que l'on peut associer à ladite composition ($C_1$), il y a tous ceux figurant dans la directive cosmétique 76/768/CEE modifiée annexe VII : notamment les composés suivants :

- le 2-phényl 5-méthyl benzoxazole, le 2,2'-hydroxy-5-méthyl phényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des polysiloxanes comme

le malonate de benzylidène siloxane ; ou ceux des familles de composés suivantes :

- Famille l'acide benzoïque comme les acides para-amino benzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Famille de l'acide anthranilique comme l'homomenthyl-N-acétyl anthranilate ;
- Famille l'acide salicylique comme le salicylate d'amyle, le salicylate d'homomenthyle, le salicylate d'éthyl hexyle, le salicylate de phényle, le salicylate de benzyle, le salicylate de p-isopropyl phényle ;
- Famille l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ;
- Famille de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 4'-phényl benzophénone-2-carboxylate de (2-éthyl hexyle), la 2-hydroxy 4-(octyloxy) benzophénone, la 4-hydroxy benzophénone-3-carboxylique ; le 3-(4'-méthylbenzylidène)-d,l-camphre, le 3-(benzylidène)-d,l-camphre, le benzalkonium méthosulfate camphre ;
- Famille de l'acide urocanique, comme cet acide ou l'urocanate d'éthyle
- Famille de l'acide sulfonique comme l'acide 2-phényl benzimidazole-5-sulfonique et ses sels ;
- Famille de la triazine comme l'hydroxyphényl triazine, l'(éthyl hexyloxy) (hydroxy) phényl 4-méthoxyphényl triazine, le 2,4,6-trianillino-(p-carbo-2'-éthyl hexyl-1'-oxy)-1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque ;
- Famille du diphénylacrylate comme le 2-éthylhexyl-2-cyano-3,3-diphényl-2-propènoate ou le l'éthyl-2-cyano-3,3-diphényl-2-propènoate ;

Parmi les filtres inorganiques solaires, également appelés "écrans minéraux", que l'on peut associer à ladite composition ($C_1$), il y a les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être optionnellement présentés sous formes de pré-dispersions aqueuses ou huileuses.

L'utilisation décrite a aussi pour objet un procédé pour améliorer l'état d'hydratation de l'épiderme de la peau humaine, caractérisé en ce qu'il comprend au moins une étape $a_1)_1$) telle que définie précédemment.

Par « amélioration de l'état d'hydratation de l'épiderme de la peau humaine », on entend au sens de la présente demande, une augmentation du taux d'hydratation du *stratum corneum* de l'épiderme de la peau humaine, mesuré et constaté dans un délai compris entre sept jours et 21 jours après l'application de ladite composition à usage topique ($C_1$), telle que définie précédemment, sur la surface dudit *stratum corneum* de l'épiderme de la peau humaine, supérieure ou égale à 25% par rapport au taux d'hydratation mesuré et constaté avant l'application de ladite composition à usage topique ($C_1$), sur la surface dudit *stratum corneum* de l'épiderme de la peau humaine à traiter.

Par « quantité efficace », on désigne, dans la définition du procédé tel que défini ci-dessus, une quantité telle que l'état d'hydratation du *stratum corneum* de l'épiderme de la peau humaine obtenu après application sur l'épiderme de la peau à traiter montre une augmentation du taux d'hydratation du *stratum corneum* de l'épiderme de la peau humaine traitée supérieure à 25% par rapport au taux d'hydratation mesuré avant l'application de la composition topique ($C_1$) telle que définie précédemment, sur la surface de l'épiderme de la peau à traiter, après une période comprise entre sept jours et vingt-un jours après l'application de ladite compositions ($C_1$) sur ladite surface de l'épiderme à traiter.

La composition ($C_1$) telle que définie précédemment est aussi décrite à titre illustratif pour son utilisation dans un traitement thérapeutique destiné à diminuer et/ou éliminer et/ou prévenir les gerçures et/ou les dartres et/ou les crevasses et/ou la dermatite atopique et/ou l'ichtyose et/ou des états de sécheresse de la peau ou des muqueuses, accompagnant des pathologies cutanées et/ou mucosales telles que l'eczéma.

La composition ($C_1$) pour son utilisation dans un traitement thérapeutique tel que défini précédemment peut être associée avec des ingrédients actifs pharmaceutiques en particulier dermatologiques.

Bibliographie :

**(1)** : Chan et al., « A review of the pharmacological effects of Arctium lappa », Inflammopharmacol, 2011, 19:245-254).
**(2)** : Rasmussen, S., Parsons, A.J., Fraser, K., Xue, H., and Newman, J.A. (2008). « Metabolic profile of Lolium Flowers and Burdock Roots using a standardized LC-DAD-ESI/MS profiling method », Journal of Agricultural and Food Chemistry 56,10105-10114.
**(3)** : Dornenburg, and Knorr (1995), « strategies secondary for improvement of metabolite production in plant cell cultures », Enzyme and Microbial Technology, 17, 674-684.
**(4)** : Dinh, P.T.Y., Roldan, M., Leung,S., and McManus, M.T (2012), « Regulation of root growth by auxin and ethylene is influenced by phosphate supply in white clover (Trifollium repensL.), Plant growth Regulation, 66, 179-180
**(5)** : Badry, D.V., and Vivanco, J.M. (2009), « Regulation and function of roots exsudatees », Plant Cell & Environment 32, 666-681.
**(6)** : Baenas N., Garcia-Viguera G., and Moreno D.A. (2014), « Elicitation : a tool for enriching the bioactive composition of foods », Molecules 19, 13541-13563.

Les exemples suivants illustrent l'invention, sans toutefois la limiter.

## $A_1$) - Préparation d'une composition ($C_{1A}$) selon l'invention.

Les plantes de Bardane ou *Arctium lappa* ont préalablement été obtenues par germination de graines pendant une durée de soixante jours dans des conditions standard, de façon à atteindre une taille d'environ dix à quinze centimètres, puis elles sont dépotées pour être placées dans des conditions de culture aéroponique consistant à laisser tremper les plantes de Bardane dans une solution nutritive caractérisée par une conductance électrique comprise entre 1,0 mS (millisiemens) et 1,2 mS et par un ratio massique N/P/K (Azote/Phosphore/Potassium) apportés par l'engrais d'environ 15/10/30 ; pendant six semaines à une température régulée à 20°C. On obtient ainsi un rendement racinaire de 754 grammes par mètre carré.
Les racines fraîches de la biomasse ainsi obtenue sont prélevées et immergées pendant quinze minutes à 25°C, à raison de 1,0 kg de biomasse par litre, dans un bain comprenant pour 100% de sa masse, 70% massique de 1,2-propanediol et 30% massique d'eau distillée dont le pH a été préalablement ajusté à 2,0 ± 0,2, par ajout d'une solution à 75% massique d'acide phosphorique.
Les plantes sont ensuite sorties de leur bain d'exsudation ($L_1$), les racines sont égouttées, puis coupées, puis remises à macérer pendant quarante-huit heures à 25°C, à raison de 0,5 kg de biomasse pour 1 litre, dans un nouveau bain comprenant un mélange comprenant pour 100% de sa masse, 70% massique de 1,2-propanediol et 30% massique d'eau distillée dont le pH a été préalablement ajusté à 2,0 ± 0,2 par ajout d'une solution à 75% massique d'acide phosphorique.
A l'issue de cette phase de macération, la biomasse est séparée du liquide de macération ($L_2$) et ledit liquide ($L_2$) est filtré avec un filtre poche de 50 micromètres pour isoler le liquide ($L_3$).
Les liquides ($L_1$) et ($L_3$) sont assemblés puis le mélange obtenu additionné de 1,2-propanediol pour en ajuster la teneur massique à 70%. Le liquide ainsi obtenu filtré sur une membrane de 1 micromètre de façon à le clarifier, puis de 0,2 micromètre pour obtenir la composition ($C_{1A}$) attendue.

## $A_2$) - Exemple de préparation d'une composition comparative ($C_{comp}$).

Les plantules issues du même lot de graines que celle utilisées pour obtenir les plantules cultivées par la suite en aéroponie (exemple $A_1$), sont utilisées pour une culture en terre desdites plantules pendant une durée de six semaines.
A l'issue de cette période, les plants sont dépotés, les racines fraîches nettoyées, coupées et broyées, puis ledit broyat obtenu est extrait selon un procédé d'extraction liquide-solide conventionnel (macération, agitation, filtration) à l'aide d'un mélange solvant 1,2-propanediol/eau distillée de 70/30, à une température de 25°C, avec un ratio massique racines fraîches/milieu solvant de 0,5 kg de biomasse pour 1 litre de de mélange 1,2-propanediol et d'eau ; la valeur du pH de l'eau distillée ayant été préalablement réglé à 2,0 ±0,2 par ajout d'une solution à 75% massique d'acide phosphorique.
A l'issue de cette phase d'extraction, la biomasse est séparée du liquide qui est ensuite filtré avec un filtre poche de 50 micromètres, puis avec une membrane de 1 micromètre de façon à le clarifier, et enfin sous filtration stérilisante avec une membrane à 0,2 micromètre, de façon à atteindre la composition ($C_{Comp}$).

## $A_3$) - Caractérisation analytique de la composition ($C_{1A}$) produit selon le procédé de l'invention et de la composition comparative ($C_{comp}$).

Les caractéristiques des compositions ($C_{1A}$) ($C_{comp}$) sont consignées dans le Tableau 11 suivant :

**Tableau 11**

| | | Composition | |
|---|---|---|---|
| Caractéristiques | Méthode analytique | ($C_{1A}$) | ($C_{comp}$) |
| Apparence | Visuelle | Liquide jaune | Liquide jaune |
| Teneur en 1,2-propanediol | CPG (espace de tête) | 71,4% | 70,0% |
| pH | Selon la norme NFT 73-206 | 3,3 | 3,2 |
| Extrait sec en % massique | Etuve à 105°C, 12 heures | 0,21% | 1,12% |
| Eau en % massique | Selon la norme NFT 73201 | 28,39% | 28,88% |
| Teneur totale en composés de formule (Ia), (Ib), ($Ic_1$) et ($Ic_2$) (mg/gramme d'extrait sec) | UPLC-MS* | 613,3 | 169,5 |
| * UPLC-MS : Appareil : Shimadzu Nexera X2 ; Colonne : Waters Xterra RP C18 ; 250x4,6 mm ; Phase mobile (avec gradient): Eau + acide formique Acétonitrile ; Détecteur UV (330 nm) | | | |

**B) Mise en évidence des propriétés hydratantes des compositions ($C_{1A}$) produit selon le procédé de l'invention et ($C_{comp}$) comparative.**

**$B_1$) Mise en évidence de la protection contre l'altération de la fonction barrière de la peau, sur des épidermes reconstruits humains.**

*B1.1. Principe de la méthode*

Les souches *Staphylococcus epidermidis* et *Staphylococcus aureus* ont été cultivées en milieux BHI (Brain Heart Infusion) et NB (Nutrient Broth) respectivement, à 37°C, pendant 24 heures. Des épidermes reconstruits humains de 0,5 $cm^2$ de surface, cultivés à 37°C et sous 5% $CO_2$, ont été colonisés tout d'abord avec la souche *Staphylococcus epidermidis* pendant une durée de 6 heures, puis ont été colonisés avec la souche *Staphylococcus aureus* pendant 24 heures.

La composition ($C_{1A}$) selon l'invention (1% v/v) a été ajoutée sur les épidermes reconstruits humains en même temps que la souche *Staphylococcus epidermidis* puis à nouveau avec la souche *Staphylococcus aureus*.

La fonction barrière des épidermes reconstruits humains ainsi traités a été évaluée :

- Par une mesure de la résistance électrique trans-épithéliale, ou TEER (Trans Epithelial Electrical Resistance), des épidermes reconstruits humains et
- Par une évaluation histologique desdits épidermes reconstruits humains, et plus particulièrement par coloration hématoxyline et éosine, et par un « score » de ladite coloration.

Dans le cadre de l'évaluation histologique, les effets sur la fonction barrière des épidermes reconstruits humains ont été évalués par un score histologique sur coloration hématoxyline et éosine qui a été attribué comme suit :

- 0 = standard : aucune modification significative de la morphologie de référence
- 1 = léger : modification significative du *stratum corneum*
- 2 = modéré : modifications significatives du *stratum corneum* et de la couche granuleuse, diminution de la kératohyaline et quelques cellules nécrotiques
- 3 = fort : modifications significatives de la couche basale avec des cellules nécrotiques et des trous intercellulaires et des oedèmes
- 4 = sévère : perte de la connexion intercellulaire, détachement du tissu du filtre polycarbonate, cellules nécrotiques, absence de marquage spécifique.

Un produit est jugé comme protecteur de la fonction barrière de l'épiderme humain si le score histologique est

noté « standard » (score 0) ou « léger » (Score 1).

L'équilibre du microbiote de l'épiderme humain reconstruit, sans ou avec application de la composition ($C_{1A}$), a été évalué par l'étude de la formation des ultrastructures type colonies, biofilms par SEM.

*B.1.2. Résultats*

*B.1.2.1 Résultats obtenus sur la protection de la fonction barrière des épidermes humains reconstruits par mesure de la résistance électrique trans-épithéliale TEER (Trans Epithelial Electrical Resistance)*

Les mesures de la TEER réalisées sur les épidermes humains reconstruits, en fonction des traitements associés sont consignées dans le Tableau 7. Une diminution de la résistance électrique trans-épithéliale (TEER) témoigne d'une dégradation de la fonction barrière de l'épiderme et par conséquent constitue un des facteurs de déshydratation de la peau et des effets inesthétiques que peut provoquer cette déshydratation. On calcule également la différence Δ entre la résistance électrique trans-épithéliale de la surface de l'épiderme humain reconstruit après colonisation et avant colonisation. On calcule également le pourcentage de protection selon la formule :

% Protection = R[Epiderme humain reconstruit colonisé avec *Staphylococcus epidermidis* + *Staphylococcus aureus* et traité par (C1A) 1% (v/v)] - R[Epiderme humain reconstruit colonisé avec *Staphylococcus epidermidis* + *Staphylococcus aureus* sans ajout de (C1A)] / R[Epiderme humain reconstruit non traité (Contrôle)] - R[Epiderme humain reconstruit colonisé avec *Staphylococcus epidermidis* + *Staphylococcus aureus* sans ajout de (C1A)]

**Tableau 7**

| **Epiderme humain reconstruit non traité (Contrôle)** | |
|---|---|
| | **A t = 0** |
| Résistance électrique (en Ohm.cm$^2$) | $R_0$ = 8073,33 +/- 1913,60 |
| | **A t = 30 heures** |
| Résistance électrique (en Ohm.cm$^2$) | $R'_0$ = 6900,00 +/- 1489,80 |
| $\Delta_0 = R'_0 - R_0$ | - 1173,33 |
| **Epiderme humain reconstruit colonisé avec *Staphylococcus epidermidis Staphylococcus* aureus sans ajout de la Composition ($C_{1A}$)** | |
| | **Avant colonisation (t = 0)** |
| Résistance électrique (en Ohm.cm$^2$) | $R_1$ = 8094,44 +/- 1486,73 |
| | **Après colonisation (t = 30 heures)** |
| Résistance électrique (en Ohm.cm$^2$) | $R'_1$ = 4726,11 +/- 2067,16 |
| $\Delta_1 = R'_1 - R_1$ | - 3368,33 |
| $(100/\Delta_0) \times (\Delta_1 - \Delta_0)$ | 187% |
| **Epiderme humain reconstruit colonisé avec *Staphylococcus epidermidis* + *Staphylococcus aureus* et traité par la Composition ($C_{1A}$) 1% (v/v) (0,0136% d'extrait sec de la composition ($C_{1A}$))** | |
| | **Avant colonisation** |
| Résistance électrique (en Ohm.cm$^2$) | $R_2$ = 7647,22 +/- 1566,61 |
| | **Après colonisation** |
| Résistance électrique (en Ohm.cm$^2$) | $R'_2$ = 5645,56 +/- 911,52 |
| $\Delta_2 = R'_2 - R_2$ | - 2001,66 |
| $(100/\Delta_0) \times (\Delta_2 - \Delta_0)$ | 71% |
| % de protection $[(R'_2 - R'_1) / (R'_0 - R'_1)]$ | 42% |

Lorsque les épidermes humains reconstruits sont colonisés avec *Staphylococcus epidermidis* et *Staphylococ-*

*cus aureus,* la différence de TEER mesurée avant et après le début de ladite colonisation est de -3368,33 Ohm.cm$^2$, et présente une augmentation de 187 % par rapport aux épidermes humains reconstruits non colonisés et non traités (-1173,33 Ohm.cm$^2$).

Lorsque les épidermes humains reconstruits sont mis en contact avec la composition ($C_{1A}$) en même temps que leur colonisation avec *Staphylococcus epidermidis* et *Staphylococcus aureus,* la différence entre les TEER mesurées avant et après ladite colonisation est de -2001,66 Ohm.cm$^2$, ce qui représente une augmentation de 71 % par rapport aux épidermes humains reconstruits non colonisés et non traités (-1173,33 Ohm.cm$^2$) et une protection de 42% par rapport aux épidermes humains reconstruits colonisés avec *Staphylococcus epidermidis* et *Staphylococcus aureus.*

Il en résulte que l'application sur la peau d'une composition comprenant la Composition ($C_{1A}$) permet de prévenir la dégradation de la fonction barrière de l'épiderme de la peau, avant que celle-ci soit soumise à l'action de bactéries connues pour leurs effets de dégradation de la fonction barrière dudit épiderme de la peau.

*B.1.2.2 Résultats obtenus sur la protection de la fonction barrière des épidermes humains reconstruits par évaluation histologique desdits épidermes reconstruits humains, par coloration hématoxyline et éosine.*

La coloration hématoxyline et éosine des épidermes a été évaluée par l'attribution de « score » tel que décrit précédemment, et les résultats sont consignés dans le tableau 8 suivant :

**Tableau 8**

| **Score histologique** |
|---|
| **Epiderme humain reconstruit non traité (Contrôle)** |
| 0 ; pas de modification significative vs morphologie de référence |
| **Epiderme humain colonisé avec *Staphylococcus* epidermidis + *Staphylococcus aureus* sans ajout de la composition ($C_{1A}$)** |
| 2 ; modification de la structure de l'épiderme viable avec plus d'espaces intercellulaires et des regroupements de cellules. |
| **Epiderme humain reconstruit colonisé avec *Staphylococcus epidermidis* + *Staphylococcus aureus* et traité avec la Composition ($C_{1A}$) 1% (v/v) (0,0136% d'extrait sec dû à la plante de la composition ($C_{1A}$))** |
| 1 ; réduction des dommages, en particulier au niveau basal et du *stratum corneum* où une structure lamellaire plus compacte est observée |

Lorsque les épidermes humains reconstruits ont été mis en contact avec la Composition ($C_{1A}$) en même temps que leur colonisation avec *Staphylococcus epidermidis* et *Staphylococcus aureus,* le score histologique a été évalué à un niveau de 1, témoignant d'une réduction des dommages, en particulier au niveau basal et du *stratum corneum* où une structure lamellaire plus compacte est observée.

Il en résulte que l'application sur la peau d'une composition comprenant la Composition ($C_{1A}$) permet de prévenir la dégradation de la cohésion tissulaire et par conséquent de la fonction barrière de l'épiderme face à une invasion de la flore transitoire.

De plus le profil de colonisation par les deux bactéries (*Staphylococcus epidermidis* et *Staphylococcus aureus*) a été évalué par microscopie électronique scannée (« Scanning electron microscopy » ou « SEM », Zeiss Sigma Electron Microscope).

Lorsque les épidermes humains reconstruits ont été colonisés par seulement *Staphylococcus epidermidis* la bactérie est présente de manière homogène à la surface de l'épiderme humain reconstruit, formant de grands agrégats et développant un biofilm caractérisé par des structures polysaccharidiques filamenteuses observé avec un grossissement de x10000 du microscope électronique.

Lorsque les épidermes humains reconstruits ont été colonisés par *Staphylococcus epidermidis* et *Staphylococcus aureus,* on observe l'apparition de plusieurs agrégats sphériques de *Staphylococcus aureus* à la surface de l'épiderme humain reconstruit, alors qu'un film de *Staphylococcus epidermidis* reste visible à la surface dudit épiderme.

On observe également la présence de grands agrégats de *Staphylococcus aureus,* avec un grossissement de x10000 du microscope électronique, formant une structure en trois dimension, indiquant ainsi un stade précoce de développement d'un biofilm à la surface de l'épiderme.

Après application sur la peau d'une composition comprenant la Composition ($C_{1A}$), les agrégats sphériques de *Staphylococcus aureus* ne sont plus présents, signifiant ainsi que l'ajout de la Composition ($C_{1A}$) permet de prévenir l'adhésion des bactéries à la surface de l'épiderme et la formation du biofilm de *Staphylococcus aureus.* Le biofilm de

*Staphylococcus epidermidis* reste toujours observé à la surface de l'épiderme.

Ces observations montrent que la Composition ($C_{1A}$) permet de réduire l'adhésion et donc la formation de biofilms de bactéries opportunistes pathogènes, comme par exemple *Staphylococcus aureus,* sans altérer la présence de bactéries commensales telles que *Staphylococcus epidermidis.*

*B.1.3. Conclusions*

La combinaison de la mesure de la résistance électrique trans-épithéliale, et de l'évaluation histologique d'épidermes reconstruits humains, avant colonisation par une bactérie commensale de la flore cutanée, puis par une bactérie pathogène, constitue un modèle permettant d'étudier l'altération de la fonction barrière et l'équilibre du microbiote dudit épiderme, et l'incidence de traitements préalables avec des compositions ou des extraits ou des formulations complexes.

L'ensemble des résultats et des observations recueillis dans les sections B.1.2.1 et B.1.2.2 montre que la composition ($C_{1A}$) permet de prévenir la dégradation de la fonction barrière de l'épiderme de la peau humaine et par conséquent de prévenir la déshydratation de l'épiderme de la peau humaine dans un modèle où elle est colonisée par une bactérie commensale de la flore cutanée, puis par une bactérie pathogène, et ses effets inesthétiques associés, par exemple une peau sèche, rugueuse, qui peut être accompagnée d'inflammation et de démangeaisons lorsqu'elle est associée à la présence de bactéries pathogènes.

**$B_2$) Mise en évidence de l'amélioration de la fonction barrière de la peau, par augmentation de la différenciation kératinocytaire**

*B.2.1 Principe de la méthode*

L'évolution de la différenciation des kératinocytes de l'épiderme constitue un moyen de montrer l'effet d'un traitement dudit épiderme sur l'évolution de la fonction barrière de l'épiderme de la peau humaine, et par conséquent sur l'évolution de l'état d'hydratation de l'épiderme de la peau humaine.

Pour cela, des kératinocytes humains normaux ont été mis en culture à 37°C et 5% $CO_2$. Après une durée de 4 jours, ils ont été traités avec les produits dont on souhaite évaluer l'action.

Après 72 heures de culture supplémentaire à 37°C et 5% $CO_2$ avec les produits à l'essai, les tapis cellulaires ont été rincés, séchés puis fixés avec du méthanol glacial. La filaggrine, marqueur de la différenciation kératinocytaire, a été détectée et quantifiée par immuno-marquage fluorescent avec un anticorps primaire anti-filaggrine et un anticorps secondaire fluorescent pour révéler ce dernier. Les noyaux ont été détectés et quantifiés par marquage au Hoechst, colorant fluorescent qui se fixe à l'ADN et donc marque les noyaux. Ce dernier marquage a été réalisé pour normaliser les résultats préalablement obtenus sur la filaggrine.

*B.2.2 Résultats*

Les mesures de la filaggrine réalisées sur les kératinocytes cultivées dans différentes conditions, et notamment en présence de la Composition ($C_{1A}$) selon l'invention et en présence de la Composition ($C_{1Com}$) comparative, sont consignées dans le Tableau 9.

Une augmentation du rapport filaggrine (aire de marquage) / noyaux (aire de marquage) témoigne d'une augmentation de la différenciation des kératinocytes de l'épiderme, et par conséquent d'un renforcement de l'effet barrière de l'épiderme, qui constitue un des facteurs de renforcement de l'état d'hydratation de la peau. Une augmentation de la différenciation kératinocytaire permet de renforcer l'effet barrière de l'épiderme humain, et par conséquent de diminuer les effets inesthétiques que peut provoquer la déshydratation de la peau humaine.

**Tableau 9 :**

| **Milieu Témoin (milieu de culture en absence de composition de traitement)** | |
|---|---|
| Filaggrine (aire de marquage) / noyaux (aire de marquage) | % stimulation vs témoin |
| 0,0034 +/- 0,0003 | - |
| **Milieu traité avec référence positive $CaCl_2$ 1mMole/L** | |
| Filaggrine (aire de marquage) / noyaux (aire de marquage) | % stimulation vs témoin |
| 0,0245 +/- 0,0045 | 621* |

(suite)

| Milieu traité avec Composition ($C_{1A}$) 0,08% massique | |
|---|---|
| Filaggrine (aire de marquage) / noyaux (aire de marquage) | % stimulation vs témoin |
| 0,0254 +/- 0,0030 | 648 |
| **Milieu traité avec Composition ($C_{1Comp}$) 0,08% massique** | |
| Filaggrine (aire de marquage) / noyaux (aire de marquage) | % stimulation vs Composition ($C_{1A}$) |
| 0,0144 +/- 0,0059 | 323p = 0,06 |

*B.2.3. Conclusions*

L'application de la Composition ($C_{1A}$) selon l'invention permet d'atteindre une valeur de ratio de filaggrine/noyau de 0,0254, contre 0,0144 lorsque la Composition ($C_{1A}$) est remplacée par la Composition ($C_{1Comp}$). Ainsi, l'application de la Composition ($C_{1A}$) selon l'invention permet d'augmenter de 648% la production de filaggrine par rapport au témoin (kératinocytes non traités) alors que la Composition ($C_{1Comp}$) comparative ne permet d'augmenter la production de filaggrine que de 323 %.

La Composition ($C_{1A}$) selon l'invention permet d'augmenter la différenciation kératinocytaire, et par conséquent de renforcer l'effet barrière de l'épiderme, et par conséquent de favoriser l'état d'hydratation dudit épiderme humain.

**$B_3$) Effet de la Composition ($C_{1A}$) sur le taux d'hydratation de la peau.**

*B.3.1. Principe de la méthode*

Le taux d'hydratation cutanée est déterminé par l'évaluation des propriétés électriques de la peau, comme par exemple l'impédance, la résistance et la capacitance, car ces paramètres diélectriques mesurés à la surface de la peau varient avec la quantité d'eau contenue dans le *stratum corneum.*

Le principe retenu et utilisé dans le cadre de la présente demande de brevet, repose sur la mesure de la variation de la capacitance diélectrique de la surface de la peau. En effet, comme tout matériau ou toute matrice biologique, le *stratum corneum* peut être caractérisé par sa valeur moyenne de capacitance ; cette propriété diélectrique varie avec la quantité d'eau qu'il contient.

*B.3.2 Matériel*

Le taux d'hydratation cutanée est mesuré à l'aide du Cornéomètre de modèle CM825™, commercialisé par la société Courage & Khasaka, équipé d'un capteur se composant de deux électrodes métalliques. Lorsque le cornéomètre est alimenté électriquement, il permet d'appliquer un champ électrique à travers le *stratum corneum* et de mesurer la capacitance correspondant à l'état de la peau sur laquelle a été appliqué le champ électrique.

*B.3.3 Protocole expérimental*

L'étude de l'effet hydratant des compositions à tester a été réalisée sur un groupe de vingt-un volontaires, et a consisté à appliquer deux fois par jour les compositions à évaluer, soit le placebo (ici nommé PLAC), soit la composition à l'essai (ici nommée COMP), soit en laissant une zone non traitée (ici nommée NT), pendant 21 jours. Les mesures du taux d'hydratation cutané mesurée à l'aide du Cornéomètre ont été réalisées avant l'application des compositions (J0), après une durée de sept jours suivant l'application des compositions à tester sur la peau (J7), et après une durée de vingt-un jours suivant l'application des compositions à tester sur la peau (J21).

Les caractéristiques du groupe de volontaires ont été les suivantes :

- femmes âgées de 18 à 48 ans,
- avec une moyenne d'âge 35 ans,
- et de phototype II à IV
- et présentant une peau très sèche au niveau des jambes (≤ 30 (ua) par cornéométrie à l'inclusion dans l'étude).

*B.3.4 Expression des résultats*

Les mesures du taux d'hydratation sont exprimées en unités arbitraires (ua).

Une augmentation des valeurs (exprimées en ua) indique une augmentation de la teneur en eau du *stratum corneum,* caractérisant ainsi un effet hydratant.

Les valeurs mesurées avec le Cornéomètre à chacun des temps de mesure sont enregistrées, et les variations entre J0 et J7, puis entre J0 et J21 sont exprimées en pourcentage par rapport à la valeur mesurée à J0, pour chaque composition à évaluer. On définit ainsi :

$T_{(J0)}$ : La valeur moyenne du taux d'hydratation, exprimée en unités arbitraires (ua), mesurée avant l'application des compositions à tester, sur la zone à traiter, et sur la zone non traitée ;

$T_{(J7)}$ : La valeur moyenne du taux d'hydratation, exprimée en unités arbitraires (ua), mesurée sept jours après l'application des compositions à tester sur la zone traitée, et sur la zone non traitée ;

$T_{(J21)}$ : La valeur moyenne du taux d'hydratation, exprimée en unités arbitraires (ua), mesurée vingt-et-un jours après l'application des compositions à tester sur la zone traitée, et sur la zone non traitée ;

On calcule ainsi $\Delta_7$, pourcentage d'augmentation du taux d'hydratation après sept jours de traitement :

Pour le placebo :

$$\Delta_7 = 100 \text{ x } [(T_{(J7)} - T_{(J0)})_{PLAC} - (T_{(J7)} - T_{(J0)})_{NT}] \, / \, [(T_{(J0)})_{PLAC} - (T_{(J7)} - T_{(J0)})_{NT}]$$

Pour la composition à l'essai :

$$\Delta_7 = 100 \text{ x } [T_{(J7)} - T_{(J0)})_{COMP} - (T_{(J7)} - T_{(J0)})_{NT}] \, / \, [(T_{(J0)})_{COMP} - (T_{(J7)} - T_{(J0)})_{NT}]$$

On calcule aussi $\Delta_{21}$, pourcentage d'augmentation du taux d'hydratation après 21 jours de traitement :

Pour le placebo :

$$\Delta_{21} = 100 \text{ x } [(T_{(J21)} - T_{(J0)})_{PLAC} - (T_{(J21)} - T_{(J0)})_{NT}] \, / \, [(T_{(J0)})_{PLAC} - (T_{(J21)} - T_{(J0)})_{NT}]$$

Pour la composition à l'essai :

$$\Delta_{21} = 100 \text{ x } [(T_{(J21)} - T_{(J0)})_{COMP} - (T_{(J21)} - T_{(J0)})_{NT}] \, / \, [(T_{(J0)})_{COMP} - (T_{(J21)} - T_{(J0)})_{NT}]$$

On calcule également l'effet entre les produits :

% vs placebo à $J_7$ =

$$100 \text{ x } (T_{(J7)} - T_{(J0)})_{COMP} - (T_{(J7)} - T_{(J0)})_{PLAC}] \, / \, [(T_{(J7)} - T_{(J0)})_{PLAC} - (T_{(J7)} - T_{(J0)})_{NT}]$$

% vs placebo à $J_{21}$ =

$$100 \text{ x } [(T_{(J21)} - T_{(J0)})_{COMP} - (T_{(J21)} - T_{(J0)})_{PLAC}] \, / \, [(T_{(J21)} - T_{(J0)})_{PLAC} - (T_{(J21)} - T_{(J0)})_{NT}]$$

*B.3.5 Résultats obtenus*

Les mesures moyennes du taux d'hydratation qui ont été obtenues pour l'application des compositions testées sont indiquées dans le tableau 10 ci-dessous :

**Tableau 10**

| Produit | $\Delta_7$ | $\Delta_{21}$ |
|---|---|---|
| Placebo | +18,3% | +22,2% |
| Formule contenant la composition ($C_{1A}$) à 1% massique | +28,8% | +37,9% |

Les comparaisons des taux d'hydratation obtenus entre le placebo et la composition à l'essai à J7 et J21 sont

indiquées dans le tableau 11 ci-dessous :

**Tableau 11**

| | % vs placébo | |
|---|---|---|
| **Produit** | **à J7** | **à J21** |
| Formule contenant la composition ($C_{1A}$) 1% massique | +58% | +56% |

*B. 3.6 Analyse des résultats*

Après 7 jours suivant l'application des compositions testées, l'évolution de la moyenne des taux d'hydratation mesurés $\Delta_7$, montre que l'augmentation du taux d'hydratation est de 28,8% pour la composition ($C_{1A}$) selon l'invention contre 18,8% pour la composition placebo. L'augmentation du taux d'hydratation de la composition ($C_{1A}$) selon l'invention est de 58% versus placebo.

Après 21 jours suivant l'application des compositions testées, l'évolution de la moyenne des taux d'hydratation mesurés $\Delta_{21}$, montre que l'augmentation du taux d'hydratation est de 37,9% pour la composition ($C_{1A}$) selon l'invention contre 22,2% pour la composition placebo. L'augmentation du taux d'hydratation de la composition ($C_{1A}$) selon l'invention est de 56% versus placebo.

Il en résulte que la composition ($C_{1A}$) produite selon le procédé de l'invention permet d'améliorer le taux d'hydratation de l'épiderme humain.

**8.4 Analyse**

L'ensemble des résultats obtenus dans la section B.3 de la présente demande, démontre que la Composition ($C_{1A}$) procure un effet hydratant sur l'épiderme de la peau humaine, tant par son rôle dans le renforcement de l'effet barrière de l'épiderme (protection contre dégradation, et augmentation) que par son action sur l'augmentation du taux d'hydratation de l'épiderme de la peau humaine.

**C) Formulations**

Dans les formules suivantes, les pourcentages sont exprimés en poids de la formulation.

**C.1) - Fluide démaquillant visage**

Formule

| | |
|---|---|
| Composition ($C_{1A}$) | 10,00% |
| Méthyl paraben | 0,15% |
| Phenoxyethanol | 0,80% |
| SEPICALM™ S | 1,00% |
| Parfum/Fragrance | 0,10% |
| Eau | q.s. 100,00% |

Mode opératoire : Mélanger les différents ingrédients dans l'eau sous agitation magnétique dans l'ordre indiqué, et ajuster le pH aux alentours de 7.

**C.2) - Shampoing cheveux et corps pour enfants**

Formule

| | | |
|---|---|---|
| A | Composition ($C_{1A}$) | 15,00% |

(suite)

| | | |
|---|---|---|
| PROTEOL™APL | | 5,00% |
| SEPICIDE™HB | | 0,50% |
| Parfum/Fragrance | | 0,10% |
| B | Eau | 20,00% |
| CAPIGEL™98 | | 3,50% |
| C | Eau | q.s. 100,00% |
| SEPICIDE™CI | | 0,30% |
| Colorant | | q.s. |
| Soude | | q.s. pH = 7,2 |
| Mode opératoire : Mélanger la composition ($E_4$) avec le PROTEOL™APL, et le SEPICIDE™HB(Phase A). Diluer le CAPIGEL™98 dans une partie de l'eau et l'ajouter à la phase A précédemment obtenue (Phase B). Ajouter le reste d'eau à la phase B, puis le SEPICIDE™CI et le colorant. Ajuster le pH du mélange à 7,2 environ avec de la soude. | | |

**C.3)** - **Lingettes démaquillantes pour les yeux**

Formule

| | | |
|---|---|---|
| A | Composition ($C_{1A}$) | 3,00% |
| B | SEPICIDE™HB2 | 0,50% |
| C | SEPICALM™ VG | 0,50% |
| Parfum/Fragrance | | 0,05% |
| D | Eau | Q.S. 100,00% |
| Mode opératoire : Mélanger les ingrédients de la phase B ainsi que ceux de la phase C dans la phase A jusqu'à obtenir la limpidité de la solution. Ajouter la phase D. | | |

**C.4)** - **Gel moussant doux**

Formule

| | | |
|---|---|---|
| A | Composition ($C_{1A}$) | 8,50% |
| PROTEOL™ APL | | 3,00% |
| EUXYL ™ PE9010 | | 1,00% |
| Parfum/Fragrance | | 0,10% |
| B | Eau | Q.S. 100,00% |
| Acide lactique | | Q.S. pH = 6,0 |
| Mode opératoire : Solubiliser le parfum et le conservateur EUXYL™ PE 9010 dans le mélange composé de la composition $E_4$ et du PROTEOL™ APL (phase A). Ajouter l'eau et régler le pH à environ 6,0 avec de l'acide lactique. | | |

**C.5)** - **Shampoing à usage fréquent**

Formule

| | | |
|---|---|---|
| A | Composition ($C_{1A}$) | 12,80% |

(suite)

| | | |
|---|---|---|
| PROTEOL™ OAT | | 5,00% |
| EUXYL ™ PE 9010 | | 1,00% |
| Parfum/Fragrance | | 0,30% |
| Eau Q.S. | | 100,00% |
| B | MONTALINE™C40 | 8,50% |
| Acide lactique | | Q.S. pH = 6,0 |

Mode opératoire : mélanger tous les ingrédients de la phase A et, après homogénéisation, ajouter la MONTALINE™C40 et ajuster le pH à environ 6,0 à l'aide de l'acide lactique.

**C.6 Shampoing ultra-doux pour bébé**

Formule

| | | |
|---|---|---|
| A | Composition ($C_{1A}$) | 10,00% |
| | AMISOFT™CS-11 | 4,00% |
| | Parfum/Fragrance | 0,10% |
| | SEPICIDE™HB | 0,30% |
| | SEPICIDE™CI | 0,20% |
| | Eau | Q.S. 100,00% |
| B | Eau | 20,00% |
| | CAPIGEL™ 98 | 3,50% |
| | Tromethamine | Q.S. pH = 7,2 |

Mode opératoire : Mélanger tous les ingrédients de la phase A dans l'ordre indiqué jusqu'à l'obtention d'une phase A limpide. De façon séparée, ajouter le CAPIGEL™98 dans l'eau, puis ajouter cette phase B ainsi préparée dans la phase A et ajuster le pH à 7,2 à l'aide de la trométhamine.

**C.7 Lait de toilette pour bébé**

Formule

| | | |
|---|---|---|
| A | SIMULSOL™165 | 2,00% |
| | MONTANOV™202 | 1,00% |
| | LANOL™99 | 3,00% |
| | Dimethicone | 1,00% |
| | Isohexadecane | 3,00% |
| B | Eau | Q.S. 100,00% |
| C | SEPIPLUS™400 | 0,30% |
| D | Composition ($C_{1A}$) | 6,35% |
| E | SEPICIDE™HB | 0,30% |
| | DMDM Hydantoin | 0,20% |
| | Parfum/Fragrance | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

### C.8 Lotion poudrée nettoyante pour peaux sensibles

Formule

| | | |
|---|---|---|
| A | LIPACIDE™C8G | 0,95% |
| | Méthyl paraben | 0,10% |
| | Ethyl paraben | 0,024% |
| | Propyl paraben | 0,0119% |
| | Butyl paraben | 0,024% |
| | Isobutyl paraben | 0,0119% |
| | Eau | 20,00% |
| | Disodium EDTA | 0,10% |
| | Triethanolamine | 1,38% |
| B | Composition ($C_{1A}$) | 1,80% |
| | Parfum/Fragrance | 0,10% |
| C | SEPICALM™S | 0,28% |
| | Eau | Q.S. 100,00% |
| | Acide lactique | Q.S. pH = 5,2 |
| D | MICROPEARL™M310 | 5,00% |

Mode opératoire : Solubiliser les ingrédients de la phase A dans l'eau à 80°C. Solubiliser séparément le parfum dans la composition ($E_4$) pour préparer la phase B. Ajouter la phase A refroidie sur la phase B, puis introduire le SEPICALM™S et le complément d'eau. Vérifier le pH final et éventuellement l'ajuster à environ 5,2. Ajouter alors le MICROPEARL™ M310.

### C.9 Gel douche enfants

Formule

| | | |
|---|---|---|
| A | Eau | 56,06% |
| | SEPIMAX™Zen | 3,00% |
| | SEPIPLUS™S | 0,80% |
| B | PROTEOL™OAT | 20,80% |
| | ORAMIX™NS 10 | 9,30% |
| | AMONYL™265 BA | 5,10% |
| C | Composition ($C_{1A}$) | 2,00% |
| | Glycéryl Glucoside | 1,00% |
| | Phenoxyéthanol & Ethylhexyl Glycérine | 1,00% |
| | Parfum/Fragrance | 0,90% |
| | Colorant | 0,04% |

Mode opératoire : disperser le SEPIMAX™ZEN dans l'eau et agiter à l'aide d'un agitateur mécanique muni d'une défloculeuse, d'une contrehélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogénéiser et ajouter individuellement les additifs de la phase C. Ajuster le pH à 6.0 - 6.5.

### C.10 BB Crème

Formule

| | |
|---|---|
| A EASYNOV™ | 2,30% |
| LANOL™ 99 | 1,00% |
| SEPIMAT™ H10W | 1,00% |
| Ethylhexyl methoxycinnamate | 5,00% |
| B Cyclométhicone | 6,00% |
| Triethoxycaprylsilane & Alumina-silane & Titanium Oxide | 8,00% |
| Iron Oxide red & Triethoxycaprylsilane | 0,24% |
| Iron Oxide yellow & Triethoxycaprylsilane | 0,66% |
| Iron Oxide black & Triethoxycaprylsilane | 0,09% |
| Parfum/Fragrance | 0,10% |
| C Eau | qs 100% |
| SEPINOV™EMT10 | 1,20% |
| D Composition ($C_{1A}$) | 2,00% |
| SEPITONIC™M3 | 1,00% |
| Phenoxyéthanol & Ethylhexyl Glycérine | 1,00% |

Mode opératoire : Préparer la phase B par mélange des différents ingrédients et homogénéiser à l'aide d'un mélangeur muni d'un système de rotor-sator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-sator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase 5 et agiter à une vitesse de 50 tours par minute pendant 25 minutes.

**C.11 Spray Solaire haute Protection SPH supérieur à 30**

Formule

| | |
|---|---|
| A MONTANOV™L | 1,00% |
| MONTANOV™82 | 1,00% |
| C12-15 Alkylbenzoate | 17,00% |
| Dimethicone | 3,00% |
| Octocrylène | 6,00% |
| Ethylhexyl methoxycinnamate | 6,00% |
| Bis-ethylhexyloxyphenol Méthoxypenyl Triazine | 3,00% |
| Tocophérol | 0,05% |
| B Eau | qs 100% |
| C SIMULGEL™INS 100 | 0,50% |
| Cyclodiméthicone | 5,00% |
| D Composition ($C_{1A}$) | 3,00% |
| Phenoxyéthanol & Ethylhexyl Glycérine | 1,00% |
| Parfum/Fragrance | 0,20% |
| E Methylene Bis-Benzotriazolyl Tetraméthylbutylphénol | 10,00% |

(suite)

| Acide citrique 25% | qs pH = 5 |
|---|---|

SEPICALM™S : Mélange de N-cocoyl aminoacides, de sarcosine, d'aspartate de potassium et d'aspartate de magnésium tel que décrit dans WO 98/09611 .
PROTEOL™APL : Mélange de sels de sodium de N-cocoyl aminoacides, obtenus par acylation des acides aminés caractéristiques du jus de pomme ;
SEPICIDE™HB : Mélange de phénoxyéthanol, de méthylparaben, d'éthylparaben, de propylparaben et de butylparaben, est un agent conservateur .
CAPIGEL™98 : Copolymère d'acrylates ;
SEPICIDE™CI : Imidazoline urée, est un agent conservateur ;
SEPICIDE™HB : Mélange de phénoxyéthanol, de méthylparaben, d'éthylparaben, de propylparaben, de butylparaben et d'isobutylparaben, est un agent conservateur ;
SEPICALM™VG : Mélange de N-palmitoyl proline sous forme de sel de sodium et d'extrait de fleur de Nymphea Alba ;
EUXYL™PE9010 : Mélange de phénoxyéthanol et d'ethyl hexyl glycérine ;
PROTEOL™OAT : Mélange de N-lauryl aminoacides obtenus par hydrolyse totale de protéine d'avoine tel que décrit dans WO 94/26694 ;
MONTALINE™C40: Sel de chlorure de Cocamidopropyl betaïnamide de Monoéthanolamine.
AMISOFT™CS-11 : Sel de sodium de N-cocoyl glutamate ;
SIMULSOL™165 : Mélange de stéarate de PEG-100 et de stéarate de glycérol ;
MONTANOV™202 (alcool arachydilique, alcool béhénique et arachidyl glucoside), est une composition auto-émulsionnable telle que celles décrites dans EP 0 977 626 ;
LANOL™99 : Isononoate d'isononyle ;
SEPIPLUS™400 : Latex inverse auto-inversible de polyacrylates dans le polyisobutene et comportant du polysorbate 20, tel que décrit dans WO2005/040230 ;
LIPACIDE™C8G : Capryloyl glycine commercialisé par la société SEPPIC ;
MICROPEARL™M310 : Polymère polyméthylméthacrylate réticulé se présentant sous forme de poudre et utilisé comme modificateur de texture ;
SEPIMAX™Zen (nom INCI : Polyacrylate Crosspolymer-6) : Polymère épaississant se présentant sous la forme d'une poudre ;
SEPIPLUS™S (nom INCI : Hydroxyethyl Acrylate / Sodium Acryloyldimethyl Taurate Copolymer & Polyisobutene & PEG-7 Trimethylolpropane Cononut Ether) : Latex inverse auto-inversible ;
AMONYL™265 BA (nom INCI : Cocobétaïne) : Agent tensioactif amphotère moussant ;
SEPINOV™EMT10 (nom INCI : Hydroxyethyl Acrylate / Sodium Acryloyldimethyl Taurate Copolymer) : Copolymère épaississant se présentant sous la forme d'une poudre ;
EASYNOV™ (nom INCI : Octyldodecanol and Octyldodecyl Xyloside and PEG-30 Dipolyhydroxystearate) : Agent émulsionnant à tendance lipophile ;
SEPIMAT™H10 FW (nom INCI :Methyl Methacrylate Crosspolymer and Squalane) : Polymère utilisé comme agent de texture ;
SEPITONIC™M3 (nom INCI : Magnesium Aspartate and Zinc Gluconate and Copper Gluconate) : Mélange utilisé comme agent antiradicalaire et énergisant pour les cellules ;
MONTANOV™L (nom INCI : C14-22 Alcohols and C12-20 Alkylglucoside) : Agent émulsionnant ;
MONTANOV™82 (nom INCI : Cetearyl Alcohol and Coco-glucoside) : Agent émulsionnant ;
SIMULGEL™INS100 (nom INCI :Hydroxyethyl Acrylate/Sodium Acryloydimethyl Taurate Copolymer and isohexadecane and Polysorbate 60) : Agent épaississant polymèrique ;

## Revendications

**1.** Procédé pour la préparation de la composition ($C_1$) comprenant pour 100% de sa masse :

**a)** - De 60,0% massique à 75,0% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol, ou d'un mélange de ces composés ;
**b)** - De 0,1% massique à 2,0% massique d'une composition (ES) comprenant une quantité massique $x_1$, exprimée en équivalent massique de l'acide 1-O-(2-caféoyl) maloyl-3,5-O-dicaféoyl quinique, supérieure ou égale à 200 mg/g d'au moins un composé de formule générale (I) :

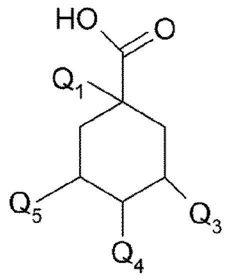

(I),

dans laquelle $Q_1$, $Q_3$, $Q_4$ et $Q_5$ représentent indépendamment les uns des autres, le radical hydroxyle ou un ses sels ou un radical choisi parmi :

**(i)** - Le radical caféoyle de formule (II) :

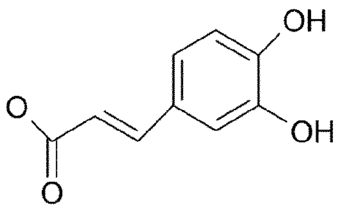

(II)

**(ii)** - Le radical maloyle de formule (IIIa) ou (IIIb) :

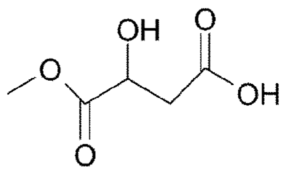

(IIIa)

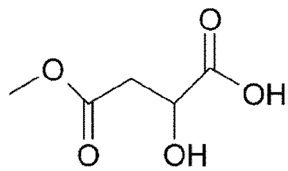

(IIIb) ;

**(iii)** - Le radical caféoyl maloyle de formule (IVa) ou (IVb) :

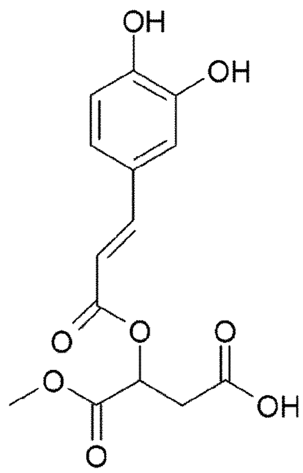

(IVa)

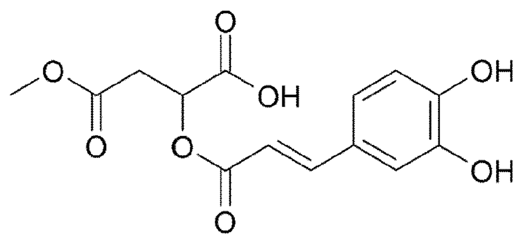

(IVb)

**(iv)** - Le radical maloyl caféoyle de formule (Va), (Vb), (Vc) ou (Vd),

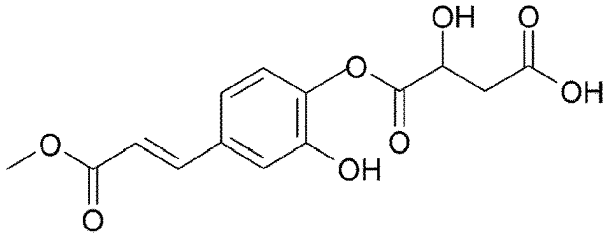

(Va)

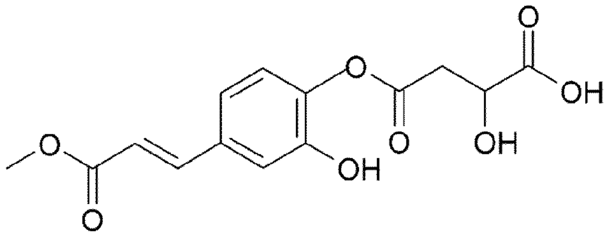

(Vb)

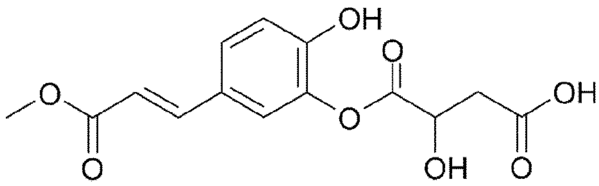

(Vc),

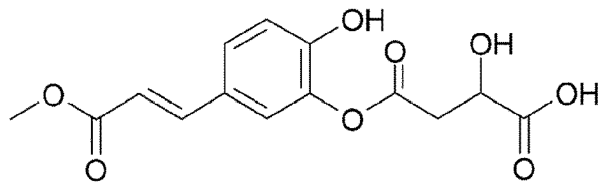

(Vd)

étant entendu qu'au moins un de ces radicaux $Q_1$, $Q_3$, $Q_4$ et $Q_5$ ne représente ni le radical -OH ; ni un de ses sels ; et

**c)** - De 20,0% massique à 35,0% massique d'eau,

ledit procédé comprenant les étapes successives suivantes :

- Une étape a) de mise en culture en conditions hors sol de la plante *Arctium lappa* alimentée par une solution nutritive, de façon à obtenir une biomasse ($BM_1$) ;
- Une étape b) d'immersion des racines de ladite biomasse ($BM_1$) obtenue à l'étape a) précédente dans un milieu ($S_1$), de telle manière que le ratio biomasse ($BM_1$)/milieu ($S_1$) soit compris entre 0,5 kg/L et 1,5 kg/L, ledit milieu ($S_1$) comprenant pour 100% de sa propre masse, de 20% à 35% massique d'eau dont le pH a été ajusté à une valeur comprise entre 1,5 et 3,5 par ajout d'un acide protique choisi parmi l'acide sulfurique, l'acide phosphorique et l'acide chlorhydrique, et de 65% à 80% massique d'un solvant organique ($SO_1$) choisi parmi le 1,2-propanediol, le 1,3-propanediol, le 1,4-butanediol, le 1,3-butanediol, le 1,2-butanediol, le 2-méthyl-2,4-pentanediol, le 1,6-hexanediol, le 1,8-octanediol ou un mélange de ces diols ;
- Une étape c) de séparation des racines de la biomasse à l'issue du traitement défini à l'étape b), pour isoler une phase liquide ($L_1$) ;
- Une étape d) d'immersion des racines de ladite biomasse ($BM_2$) issue de l'étape c) dans ledit milieu ($S_1$); dans un ratio biomasse ($BM_2$)/milieu ($S_1$) compris entre 0,1 kg/L et 1,5 kg/L ;
- Une étape e) de séparation de ladite biomasse ($BM_2$) à l'issue du traitement défini à l'étape d), pour isoler une phase liquide ($L_2$),
- Une étape f) de filtration de ladite phase liquide ($L_2$) obtenue à l'étape e), pour isoler une phase liquide ($L_3$),
- Une étape g) de mélange des dites phases liquides ($L_1$) et ($L_3$), puis si nécessaire d'addition d'eau et/ou dudit

solvant organique ($SO_1$), de façon à obtenir la composition ($C_1$) attendue.

**2.** Procédé tel que défini à la revendication 1 **caractérisé en ce que** ladite composition (ES) comprend :

- Au moins un composé de formule (la) correspondant à la formule (I) pour laquelle $Q_1$ représente le radical maloyle de formule (IIIa) ou de formule (IIIb) et $Q_3$ et $Q_4$ et $Q_5$ identiques, représentent chacun le radical caféoyle de formule (II) ;
- Un composé de formule (Ib) correspondant à la formule (I) pour laquelle $Q_1$ représente le radical caféoylmaloyle de formule (IVa) ou de formule (IVb), $Q_3$ et $Q_5$ représentent chacun le radical caféoyle de formule (II) et $Q_4$ représente le radical hydroxyle, et
- Au moins un composé de formule (Ic) choisi parmi :

    - Le composé de formule ($Ic_1$) correspondant à la formule (I) pour laquelle $Q_1$ et $Q_5$ représentent chacun le radical caféoyle de formule (II), $Q_3$ représente le radical hydroxyle et $Q_4$ représente le radical caféoylmaloyle de formule (IVa) ou de formule (IVb) ; et
    - Le composé de formule ($Ic_2$) correspondant à la formule (I) pour laquelle $Q_1$ et $Q_4$ représentent le radical caféoyle de formule (II), $Q_3$ représente le radical hydroxyle et $Q_5$ représente le radical caféoylmaloyle de formule (IVa) ou de formule (IVb).

**3.** Procédé tel que défini à l'une ou quelconque des revendications 1 ou 2 **caractérisée en ce que** le solvant organique ($SO_1$) est le1,2-propanediol.

**Patentansprüche**

**1.** Verfahren zur Herstellung der Zusammensetzung ($C_1$), die zu 100 % ihrer Masse umfasst:

**a)** - 60,0 Gew.-% bis 75,0 Gew.-% eines organischen Lösungsmittels ($SO_{1)}$, ausgewählt aus 1,2-Propandiol, 1,3-Propandiol, 1,4-Butandiol, 1,3-Butandiol, 1,2-Butandiol, 2-Methyl-2,4-pentandiol, 1,6-Hexandiol, 1,8-Octandiol oder einem Gemisch dieser Verbindungen;
**b)** - 0,1 Gew-% bis 2,0 Gew-% einer Zusammensetzung (ES), die eine Gew.-Menge $x_1$, ausgedrückt als Gewichtsäquivalent von 1-O-(2-Caffeoyl)-maloyl-3,5-O-dicaffeoylchinasäure, von größer oder gleich 200 mg/g von mindestens einer Verbindung der allgemeinen Formel (I) umfasst:

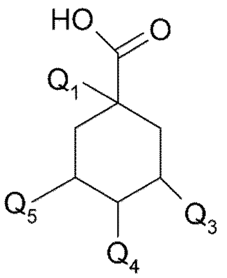

(I),

worin $Q_1$, $Q_3$, $Q_4$ und $Q_5$ unabhängig voneinander den Hydroxylrest oder ein Salz davon oder einen Rest darstellen, der ausgewählt ist aus:

**(i)** - Der Caffeoylrest der Formel (II) :

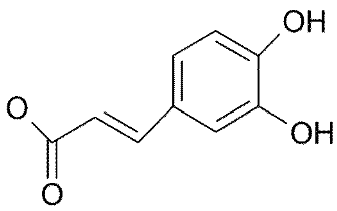

(II)

**(ii)** - Der Maloylrest der Formel (IIIa) oder (IIIb) :

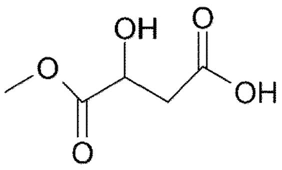
(IIIa)
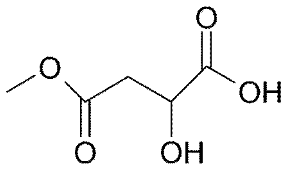
(IIIb) ;

**(iii)** - dem Caffeoylmaloylrest der Formel (IVa) oder (IVb) :

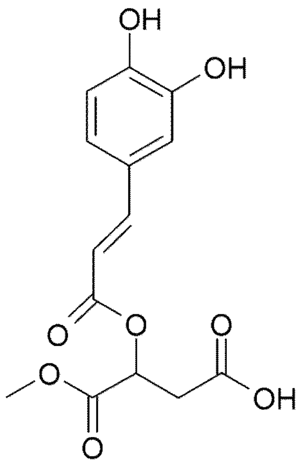
(IVa)
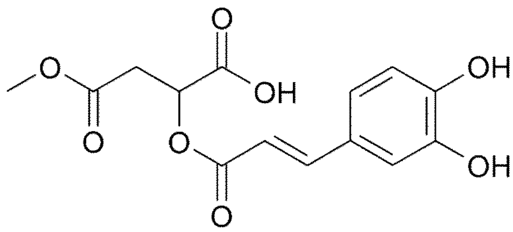
(IVb)

**(iv)** - Der Maloylcaféoylrest der Formel (Va), (Vb), (Vc) oder (Vd),

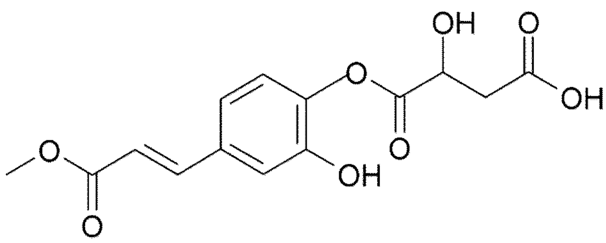
(Va)

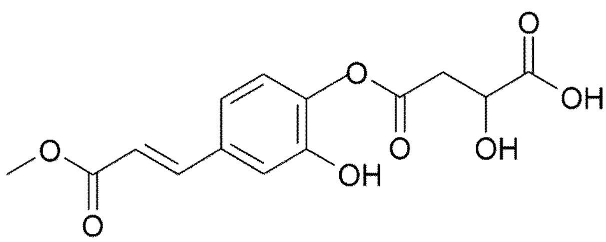
(Vb)

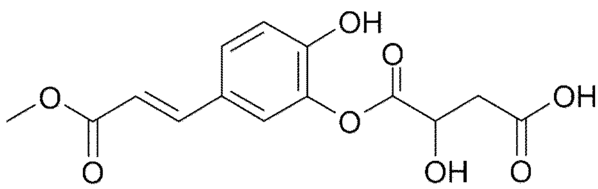
(Vc),

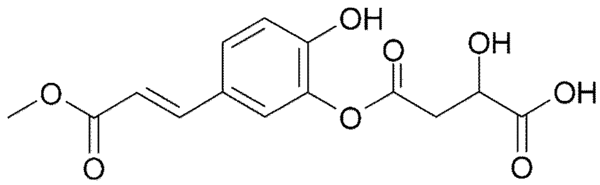

(Vd)

mit der Maßgabe, dass mindestens einer dieser Reste $Q_1$, $Q_3$, $Q_4$ und $Q_5$ weder den Rest -OH darstellt; noch eines seiner Salze; und
c) - Von 20,0 Massenprozent bis 35,0 Massenprozent Wasser,

wobei das Verfahren die folgenden aufeinanderfolgenden Schritte umfasst:

- Ein Schritt a) des Kultivierens der *Arctium lappa-Pflanze,* die mit einer Nährlösung versorgt wird, unter bodenlosen Bedingungen, um eine Biomasse ($BM_1$) zu erhalten ;
- Ein Schritt b) des Eintauchens der Wurzeln der im vorhergehenden Schritt a) erhaltenen Biomasse ($BM_1$) in ein Medium ($S_1$), so dass das Verhältnis Biomasse ($BM_1$)/Medium ($S_1$) zwischen 0,5 kg/L und 1,5 kg/L liegt, wobei das Medium ($S_1$) zu 100 % seiner eigenen Masse 20 bis 35 Gew-% Wasser umfasst, dessen pH-Wert auf einen Wert zwischen 1,5 und 3 eingestellt wurde,5 durch Zugabe einer protischen Säure, ausgewählt aus Schwefelsäure, Phosphorsäure und Salzsäure, eingestellt wurde, und 65% bis 80% Masseanteil eines organischen Lösungsmittels ($SO_1$), ausgewählt aus 1,2-Propandiol, 1,3-Propandiol, 1,4-Butandiol, 1,3-Butandiol, 1,2-Butandiol, 2-Methyl-2,4-pentandiol, 1,6-Hexandiol, 1,8-Octandiol oder einer Mischung dieser Diole ;
- Ein Schritt c) zum Abtrennen der Wurzeln von der Biomasse nach der in Schritt b) definierten Behandlung, um eine flüssige Phase ($L_1$) zu isolieren ;
- Ein Schritt d) Eintauchen der Wurzeln der Biomasse ($BM_2$) aus Schritt c) in das Medium ($S_1$) bei einem Verhältnis von Biomasse ($BM_2$)/Medium ($S_1$) zwischen 0,1 kg/L und 1,5 kg/L ;
- Ein Schritt e) zum Trennen der Biomasse ($BM_2$) nach der in Schritt d) definierten Behandlung, um eine flüssige Phase ($L_2$) zu isolieren,
- Ein Schritt f) des Filterns der flüssigen Phase ($L_2$), die in Schritt e) erhalten wurde, um eine flüssige Phase ($L_3$) zu isolieren,
- Ein Schritt g) Mischen der flüssigen Phasen ($L_1$) und ($L_3$), dann, falls nötig, Zugabe von Wasser und/oder des organischen Lösungsmittels ($SO_1$), um die erwartete Zusammensetzung ($C_1$) zu erhalten.

**2.** Verfahren nach Anspruch 1, **dadurch gekennzeichnet, dass** die Zusammensetzung (ES) umfasst :

- Mindestens eine Verbindung der Formel (Ia), die der Formel (I) entspricht, in der $Q_1$ den Maloylrest der Formel (IIIa) oder der Formel (IIIb) darstellt und $Q_3$ und $Q_4$ und $Q_5$, die identisch sind, jeweils den Cafeoylrest der Formel (II) darstellen;
- Eine Verbindung der Formel (Ib), die der Formel (I) entspricht, in der $Q_1$ den Caffeoylmaloylrest der Formel (IVa) oder der Formel (IVb) darstellt, $Q_3$ und $Q_5$ jeweils den Caffeoylrest der Formel (II) darstellen und $Q_4$ den Hydroxylrest darstellt, und
- Mindestens eine Verbindung der Formel (Ic), ausgewählt aus:

  - Die Verbindung der Formel ($Ic_1$), die der Formel (I) entspricht, in der $Q_1$ und $Q_5$ jeweils den Caffeoylrest der Formel (II) darstellen, $Q_3$ den Rest Hydroxyl darstellt und $Q_4$ den Caffeoylmaloylrest der Formel (IVa) oder der Formel (IVb) darstellt; und
  - Die Verbindung der Formel ($Ic_2$), die der Formel (I) entspricht, in der $Q_1$ und $Q_4$ den Caffeoylrest der Formel (II) darstellen, $Q_3$ den Hydroxylrest darstellt und $Q_5$ den Caffeoylmaloylrest der Formel (IVa) oder der Formel (IVb) darstellt.

**3.** Verfahren gemäß einer der Ansprüche 1 oder 2, **dadurch gekennzeichnet, dass** das organische Lösungsmittel ($SO_1$) 1,2-Propandiol ist.

## Claims

**1.** Process for the preparation of the composition ($C_1$) comprising, for 100% of its mass:

**a)** - from 60.0% by weight to 75.0% by weight of an organic solvent ($SO_1$) chosen from 1,2-propanediol, 1,3-propanediol, 1,4-butanediol, 1,3-butanediol, 1,2-butanediol, 2-methyl-2,4-pentanediol, 1,6-hexanediol, 1,8-octanediol, or a mixture of these compounds;
**b)** - from 0.1% by mass to 2.0% by mass of a composition (ES) comprising a quantity by mass $x_1$, expressed as equivalent by mass of 1-O-(2-caffeoyl)maloyl-3,5-O-dicaffeoylquinic acid, greater than or equal to 200 mg/g of at least one compound of general formula (I):

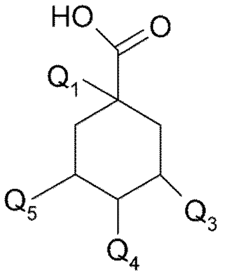

(I),

in which $Q_1$, $Q_3$, $Q_4$ and $Q_5$ independently of one another represent the hydroxyl radical or a salt thereof or a radical chosen from :

**(i)** - The caffeoyl radical of formula (II) :

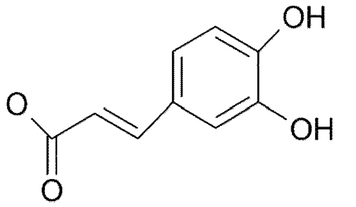

(II)

**(ii)** - The maloyl radical of formula (IIIa) or (IIIb) :

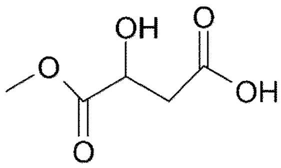

(IIIa)

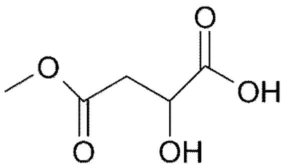

(IIIb) ;

**(iii)** - The caffeoyl maloyl radical of formula (IVa) or (IVb) :

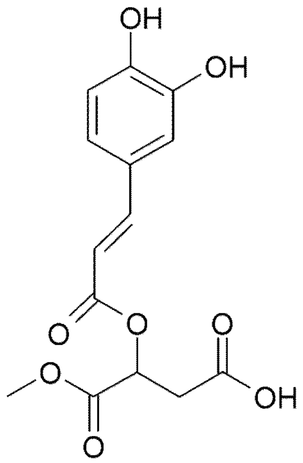

(IVa)

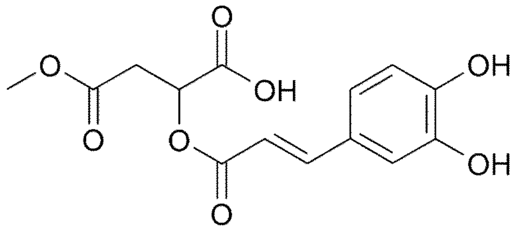

(IVb)

**(iv)** - The maloyl caffeoyl radical of formula (Va), (Vb), (Vc) or (Vd),

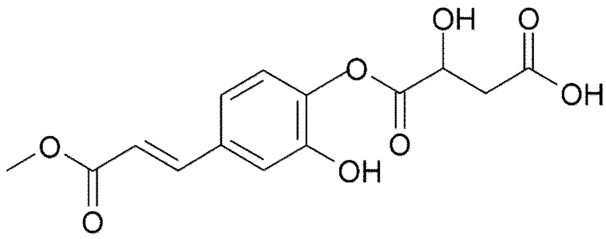

(Va)

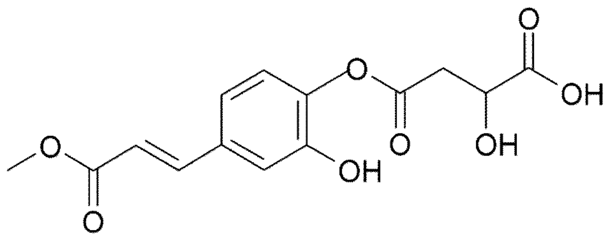

(Vb)

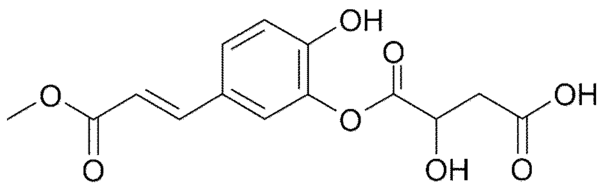

(Vc),

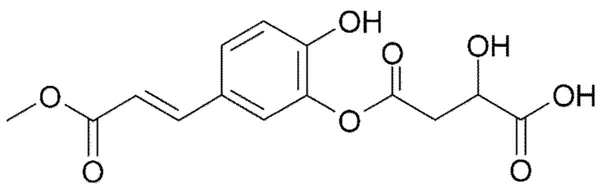

(Vd)

it being understood that at least one of these radicals $Q_1$, $Q_3$, $Q_4$ and $Q_5$ represents neither the radical -OH; nor one of its salts; and

c) - From 20.0% by mass to 35.0% by mass of water,

said process comprising the following successive steps:

- A step a) of growing the *Arctium lappa* plant in soilless conditions, fed with a nutrient solution, so as to obtain a biomass ($BM_1$) ;
- A step b) of immersing the roots of the said biomass ($BM_1$) obtained in the preceding step a) in a medium ($S_1$), in such a way that the biomass ($BM_1$)/medium ($S_1$) ratio is between 0.5 kg/L and 1.5 kg/L, the said medium ($S_1$) comprising, for 100% of its own mass, from 20% to 35% by mass of water, the pH of which has been adjusted to a value between 1.5 and 3,5 by addition of a protic acid chosen from sulphuric acid, phosphoric acid and hydrochloric acid, and from 65% to 80% by weight of an organic solvent ($SO_1$) chosen from 1,2-propanediol, 1,3-propanediol, 1,4-butanediol, 1,3-butanediol, 1,2-butanediol, 2-methyl-2,4-pentanediol, 1,6-hexanediol, 1,8-octanediol or a mixture of these diols;
- A step c) for separating the roots from the biomass at the end of the treatment defined in step b), in order to isolate a liquid phase ($L_1$);
- A step d) of immersing the roots of said biomass ($BM_2$) from step c) in said medium ($S_1$); in a biomass ($BM_2$)/medium ($S_1$) ratio of between 0.1 kg/L and 1.5 kg/L;
- A step e) of separating said biomass ($BM_2$) after the treatment defined in step d), to isolate a liquid phase ($L_2$),
- A step f) of filtering said liquid phase ($L_2$) obtained in step e), to isolate a liquid phase ($L_3$),
- A step g) of mixing the said liquid phases ($L_1$) and ($L_3$), then if necessary adding water and/or the said organic solvent ($SO_1$), to obtain the expected composition ($C_1$).

**2.** Process as defined in claim 1, **characterized in that** the said composition (ES) comprises:

- At least one compound of formula (Ia) corresponding to formula (I) for which $Q_1$ represents the maloyl radical of formula (IIIa) or of formula (IIIb) and $Q_3$ and $Q_4$ and $Q_5$, which are identical, each representing the caffeoyl radical of formula (II) ;
- A compound of formula (Ib) corresponding to formula (I) in which $Q_1$ represents the caffeoylalkyl radical of formula (IVa) or of formula (IVb), $Q_3$ and $Q_5$ each represents the caffeoyl radical of formula (II) and $Q_4$ represents the hydroxyl radical, and
- At least one compound of formula (Ic) chosen from:
  - The compound of formula ($Ic_1$) corresponding to formula (I) for which $Q_1$ and $Q_5$ each represent the caffeoyl radical of formula (II), $Q_3$ represents the hydroxyl radical and $Q_4$ represents the caffeoylmalkyl radical of formula (IVa) or of formula (IVb); and
  - The compound of formula ($Ic_2$) corresponding to formula (I) for which $Q_1$ and $Q_4$ represent the caffeoyl radical of formula (II), $Q_3$ represents the hydroxyl radical and $Q_5$ represents the caffeoylmalkyl radical of formula (IVa) or of formula (IVb).

**3.** Process as defined in any one of claims 1 or 2, **characterized in that** the organic solvent (SO,) is 1,2-propanediol.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CN 104304955 A
- CN 105232438 A
- CN 107157800 A
- CN 106074663 A
- WO 9809611 A
- WO 9426694 A
- EP 0977626 A
- WO 2005040230 A


### Littérature non-brevet citée dans la description


- Dictionary International Cosmetic Ingredient Handbook / Natural Solution. *INTERNATIONAL COSMETIC INGREDIENT DICTIONARY AND HANDBOOK* (88), 5
- **CHAN et al.** A review of the pharmacological effects of Arctium lappa. *Inflammopharmacol*, 2011, vol. 19, 245-254
- **RASMUSSEN, S.** ; **PARSONS, A.J** ; **FRASER, K.** ; **XUE, H** ; **NEWMAN, J.A**. Metabolic profile of Lolium Flowers and Burdock Roots using a standardized LC-DAD-ESI/MS profiling method. *Journal of Agricultural and Food Chemistry*, 2008, vol. 56, 10105-10114
- **DORNENBURG** ; **KNORR**. strategies secondary for improvement of metabolite production in plant cell cultures. *Enzyme and Microbial Technology*, 1995, vol. 17, 674-684
- **DINH, P.T.Y** ; **ROLDAN, M** ; **LEUNG,S** ; **MCMANUS, M.T**. Regulation of root growth by auxin and ethylene is influenced by phosphate supply in white clover (Trifollium repensL.). *Plant growth Regulation*, 2012, vol. 66, 179-180
- **BADRY, D.V** ; **VIVANCO, J.M.** Regulation and function of roots exsudatees. *Plant Cell & Environment*, 2009, vol. 32, 666-681
- **BAENAS N** ; **GARCIA-VIGUERA G.** ; **MORENO D.A.** Elicitation : a tool for enriching the bioactive composition of foods. *Molecules*, 2014, vol. 19, 13541-13563